# EUROPÄISCHE PATENTSCHRIFT

(11) **EP 4 460 277 B1**
(45) Veröffentlichungstag und Bekanntmachung des Hinweises auf die Patenterteilung: **10.12.2025**
(21) Anmeldenummer: 23700582.2
(22) Anmeldetag: 03.01.2023
(51) Int. Cl.: A61F 9/008

(54) **VERFAHREN ZUR BEREITSTELLUNG EINER SOLL-STRAHLFÜHRUNGSBAHN UND OPHTHALMISCHES LASERSYSTEM**
METHOD FOR THE PROVISION OF A TARGET BEAM GUIDANCE PATH AND OPHTHALMIC LASER SYSTEM
PROCÉDÉ POUR FOURNIR UNE VOIE DE GUIDAGE DE FAISCEAU DE CONSIGNE ET SYSTÈME DE LASER OPHTALMOLOGIQUE

(30) Priorität: 07.01.2022 DE 102022200119
(43) Veröffentlichungstag der Anmeldung: 13.11.2024
(73) Patentinhaber: Carl Zeiss Meditec AG, 07745 Jena (DE)
(72) Erfinder: WEIMER, Wolf, 07745 Jena (DE); WEYHAUSEN, Andreas, 07745 Jena (DE)
(74) Vertreter: Tautz & Schuhmacher
(86) Internationale Anmeldenummer: PCT/EP2023/050049
(87) Internationale Veröffentlichungsnummer: WO 2023/131601

(56) Entgegenhaltungen:
- WO-A1-2010/136050
- US-A1- 2018 214 305

## Beschreibung

Bereitgestellt werden ein Verfahren zur Bereitstellung einer Soll-Strahlführungsbahn zur Ablenkung eines Laserstrahls eines ophthalmologischen Lasersystems mittels einer Verstelleinrichtung, eine Steuereinheit, ein ophthalmologisches Lasersystem, ein Computerprogrammprodukt und ein computerlesbares Speichermedium. Die Ausführungsformen liegen insbesondere auf dem Gebiet der Lasersysteme für refraktive Chirurgie.

Ophthalmologische Lasersysteme weisen oftmals eine Verstelleinrichtung auf, mittels welcher der Laserstrahl und insbesondere die Fokuslage relativ zum zu behandelnden Objekt, wie etwa ein zu behandelndes Auge, variiert werden kann. Dazu kann die Verstelleinrichtung derart gesteuert und/oder geregelt werden, dass der Laserstrahl und/oder der Fokus senkrecht zur Ausbreitungsrichtung des Laserstrahls bewegt werden kann und/oder die Fokuslage entlang der Ausbreitungsrichtung des Laserstrahls variiert werden kann.

Die Verstelleinrichtungen verursachen herkömmlicherweise einen Losbrechsprung, wenn diese aus dem Stillstand in Bewegung versetzt werden. Diese Problematik tritt insbesondere bei Linearverstellern auf, wobei auch sogenannte Galvo-Scanner davon betroffen sein können. Beispielsweise können demnach Losbrechsprünge bei als Linearverstellern ausgebildeten Verstelleinrichtungen auftreten, welche beispielsweise als z-Scanner, d.h. entlang der Strahlrichtung des Laserstrahls, in ophthalmologischen Lasersystemen Verwendung finden können, während hingegen ein x-y-Scanner (in einer Ebene senkrecht zur Strahlrichtung des Laserstrahls) als Galvo-Scanner ausgebildet sein kann, welcher in geringerem Maße von Losbrechsprüngen betroffen sein kann. Alternativ kann jedoch ein Linearversteller auch als x-y--Scanner zum Einsatz kommen. Auch dann treten die Losbrechsprünge insbesondere im x-y-Scanner auf. Losbrechsprünge können insbesondere dann auftreten, wenn die Verstelleinrichtung (kurzzeitig) zum Stillstand kommt und anschließend wieder in Bewegung versetzt wird. Zu diesem Zweck wird die Verstelleinrichtung typischerweise mit einem Steuersignal zur Positionierung an einer Soll-Position beaufschlagt, dem die Verstelleinrichtung nur dann folgt, wenn die Abweichung zu seiner Ist-Position zur Soll-Position eine gewisse Sprunggröße überschreitet. Die Verstelleinrichtung bleibt demnach eine kurze Zeit stehen und akkumuliert einen größeren Positionsfehler bevor sie sprungartig die Sollposition zu erreichen versucht. Wenn während der Zeitspanne mit dem kumulierten Positionsfehler der Laserstrahl aktiviert ist, kann dies zu ungenauen Schnitten in dem zu behandelnden Objekt, beispielsweise in der Cornea eines zu behandelnden Auges, oder gar einem sicherheitsbedingten Behandlungsabbruch durch eine technische Fehlerüberwachung führen.

Die US 2018/0214305 A1 beschreibt ein Laseroperationsverfahren, bei welchem der Laserstrahl mäanderförmig über die Behandlungszone geführt wird, wobei außerhalb der Behandlungszone die Bewegungsrichtung des Laserstrahls umgekehrt wird.

Die EP 0770370 A2 beschreibt ein Scanverfahren für eine Laseroperationsvorrichtung, bei welcher in einem rechteckigen Rasterscan der Laserstrahl über die Behandlungszone bewegt wird.

Die WO 2013/053366 A1 beschreibt einen mäanderförmigen Scanpfad, bei welchem die Bewegungsrichtung außerhalb des Behandlungsbereichs umgekehrt wird.

Weiterhin beschreibt die WO 2010/136050 ein System für die laserchirurgische Ophthalmologie, umfassend ein Lasersystem mit auf die Anbringung einer Inzision in einem Augengewebe abgestimmten Strahlungsparametern, umfassend einen Scanner, eine Modulatoreinheit und eine Steuereinheit. Die Steuereinheit ist dabei dazu eingerichtet, die Modulatoreinheit nach Maßgabe eines für die Schnittgeometrie festgelegten Strahlablenkungsmusters derart zu steuern, dass in vorbestimmten Teilen des Strahlablenkungsmusters mindestens ein Teil der Laserpulse eine verminderte Pulsenergie besitzt oder unterdrückt ist.

Es ist daher wünschenswert, dass etwaig erforderliche Stillstände der Verstelleinrichtung in Behandlungsphasen mit deaktiviertem Laser gelegt werden. In diesen Phasen mit deaktiviertem Laser kann sodann nach dem Ende einer vorherigen Schnittsequenz bzw. nach einem vorhergehenden Bestrahlungsabschnitt der Laserstrahl neu positioniert werden, um an der gewünschten Position mit der nächsten Schnittsequenz bzw. dem nächsten Bestrahlungsabschnitt zu beginnen. Aufgrund des Losbrechsprungs und der dadurch verursachten abrupten Bewegung der Verstelleinrichtung beim Überführen der Verstelleinrichtung aus dem Stillstand in eine kontinuierliche Bewegung tritt jedoch herkömmlicherweise auch während der darauffolgenden Schnittsequenz und dem darauffolgenden Bestrahlungsabschnitt eine Abweichung von der beabsichtigten Strahlführung auf, welche die Genauigkeit der Bestrahlung bzw. Behandlung reduziert.

Insbesondere bei der Erzeugung von gekrümmten Schnittflächen, etwa bei einer Durchführung einer refraktiven chirurgischen Behandlung gemäß der SMILE Behandlungsart (SMILE = small incision lenticle extration) oder ähnlichen chirurgischen Eingriffen, bei denen komplexe Strukturen in der Hornhaut erzeugt werden, können mehrere Stillstände der Verstelleinrichtung erforderlich sein, sodass mehrere Losbrechsprünge auftreten können und das Problem entsprechend mehrfach während einer Behandlung auftreten kann. Ebenso kann dies beim Erzeugen zusammengesetzter Schnittmuster mehrfach auftreten, da mehrere einzelne Schnitte durchgeführt werden müssen und die Verstelleinrichtung zwischen den einzelnen Schnitten zum Stillstand kommt.

Herkömmlicherweise versucht man auftretenden Abweichungen durch eine Echtzeitfehlerüberwachung im ophthalmologischen Lasersystem zu begegnen, welche bei Überschreiten eines Grenzwertes hinsichtlich des Fehlers die Behandlung abbricht. Dies kann zwar eine Fehlbehandlung durch eine zu starke Abweichung von geplanten Schnitten verhindern, führt jedoch zu abgebrochenen Schnitten oder gar zu einer nicht durchführbaren oder nicht weiterführbaren Behandlung.

Ein anderer herkömmlicher Ansatz umfasst eine Verringerung der Geschwindigkeit bei der Schnittführung, welche eine Erhöhung der Präzision ermöglicht, aber den Nachteil einer entsprechend langen Behandlungsdauer und damit einhergehenden Problemen mit sich bringt.

Es liegt daher die Aufgabe zugrunde, die Präzision und Zuverlässigkeit von ophthalmologischen Lasersystemen auch bei hohen Schnittgeschwindigkeiten zu verbessern.

Die Aufgabe wird gelöst durch ein Verfahren zur Bereitstellung einer Soll-Strahlführungsbahn zur Ablenkung eines Laserstrahls eines ophthalmologischen Lasersystems mittels einer Verstelleinrichtung, eine Steuereinheit, ein ophthalmologisches Lasersystem, ein Computerprogrammprodukt und ein computerlesbares Speichermedium mit den Merkmalen der jeweiligen unabhängigen Ansprüche. Optionale Ausgestaltungen sind in den Unteransprüchen und in der Beschreibung angegeben.

Eine erste Ausführungsform betrifft Verfahren zur Bereitstellung einer Soll-Strahlführungsbahn zur Ablenkung eines Laserstrahls eines ophthalmologischen Lasersystems mittels einer Verstelleinrichtung. Das Verfahren umfasst ein Festlegen eines Anpassungsabschnitts der Soll-Strahlführungsbahn, in welchem die Verstelleinrichtung zur Strahlführung des Laserstrahls aus dem Stillstand an einem Stillstandspunkt in eine kontinuierliche Bewegung überführt wird und in welchem keine Bestrahlung eines zu behandelnden Objektes mit dem Laserstrahl vorgesehen ist. Zudem umfasst das Verfahren ein Festlegen eines Bestrahlungsabschnitts der Soll-Strahlführungsbahn, welcher an den Anpassungsabschnitt angrenzt, in welchem mittels der Verstelleinrichtung der Laserstrahl eine kontinuierliche Bewegung durchführt und in welchem eine Bestrahlung eines zu behandelnden Objektes mittels des Lasersystems vorgesehen ist. Dabei erfolgt das Festlegen einer Länge und/oder Zeitdauer des Anpassungsabschnitts und/oder eines Abstands des Beginns des Bestrahlungsabschnitts vom Stillstandspunkt des Anpassungsabschnitts derart, dass eine mit der Überführung der Verstelleinrichtung aus dem Stillstand in die kontinuierliche Bewegung einhergehende Abweichung einer Ist-Strahlführungsbahn von der festgelegten Soll-Strahlführungsbahn im gesamten Bestrahlungsabschnitt kleiner als ein vorbestimmter Grenzwert ist.

Eine weitere Ausführungsform betrifft eine Steuereinheit zur Bereitstellung einer Soll-Strahlführungsbahn zur Ablenkung eines Laserstrahls eines ophthalmologischen Lasersystems mittels einer Verstelleinrichtung. Die Steuereinheit ist dazu eingerichtet, einen Anpassungsabschnitt der Soll-Strahlführungsbahn festzulegen, in welchem die Verstelleinrichtung zur Strahlführung des Laserstrahls aus dem Stillstand an einem Stillstandspunkt in eine kontinuierliche Bewegung überführt wird und in welchem keine Bestrahlung eines zu behandelnden Objektes mit dem Laserstrahl vorgesehen ist. Ferner ist die Steuereinheit dazu eingerichtet, einen Bestrahlungsabschnitt der Soll-Strahlführungsbahn festzulegen, welcher an den Anpassungsabschnitt angrenzt, in welchem mittels der Verstelleinrichtung der Laserstrahl eine kontinuierliche Bewegung durchführt und in welchem eine Bestrahlung eines zu behandelnden Objektes mittels des Lasersystems vorgesehen ist. Zudem ist die Steuereinheit dazu eingerichtet, eine Länge und/oder Zeitdauer des Anpassungsabschnitts und/oder eines Abstands des Beginns des Bestrahlungsabschnitts vom Stillstandspunkt des Anpassungsabschnitts derart festzulegen, dass eine mit der Überführung der Verstelleinrichtung aus dem Stillstand in die kontinuierliche Bewegung einhergehende Abweichung einer Ist-Strahlführungsbahn von der festgelegten Soll-Strahlführungsbahn im gesamten Bestrahlungsabschnitt kleiner als ein vorbestimmter Grenzwert ist.

Eine weitere Ausführungsform betrifft ein ophthalmologisches Lasersystem. Das Lasersystem umfasst eine Laserquelle zur Bereitstellung eines Laserstrahls zur Behandlung eines zu behandelnden Objektes, sowie eine Verstelleinrichtung zur Strahlführung des Laserstrahls gemäß einer vorgegebenen Soll-Strahlführungsbahn, wobei die Verstelleinrichtung bei einer Überführung aus dem Stillstand an einem Stillstandspunkt in eine kontinuierliche Bewegung einen Losbrechsprung verursacht. Außerdem umfasst das Lasersystem eine Steuereinheit zur Festlegung der Soll-Strahlführungsbahn zur Bereitstellung für die Verstelleinrichtung, wobei die Steuereinheit dazu eingerichtet ist, einen Anpassungsabschnitt der Soll-Strahlführungsbahn festzulegen, in welchem die Verstelleinrichtung zur Strahlführung des Laserstrahls aus dem Stillstand an dem Stillstandspunkt in eine kontinuierliche Bewegung überführt wird und in welchem keine Bestrahlung eines zu behandelnden Objektes mit dem Laserstrahl erfolgt, sowie zur Festlegung eines Bestrahlungsabschnitts der Soll-Strahlführungsbahn festzulegen, welcher an den Anpassungsabschnitt angrenzt, in welchem mittels der Verstelleinrichtung der Laserstrahl eine kontinuierliche Bewegung durchführt und in welchem eine Bestrahlung des zu behandelnden Objektes mit dem Laserstrahl erfolgt. Ferner umfasst das ophthalmologische Lasersystem eine Überwachungseinheit zur Ermittlung einer Ist-Strahlführungsbahn entlang welcher die Strahlführung des Laserstrahls mittels der Verstelleinrichtung erfolgt. Dabei ist das ophthalmologisches Lasersystem dazu eingerichtet, eine mit dem Losbrechsprung einhergehende Abweichung der Ist-Strahlführungsbahn von der Soll-Strahlführungsbahn zu ermitteln und eine Länge und/oder Zeitdauer des Anpassungsabschnitts und/oder eines Abstands des Beginns des Bestrahlungsabschnitts vom Stillstandspunkt des Anpassungsabschnitts derart festzulegen, dass die Abweichung der Ist-Strahlführungsbahn von der Soll-Strahlführungsbahn im gesamten Bestrahlungsabschnitt kleiner als ein vorbestimmter Grenzwert ist.

Eine weitere Ausführungsform betrifft ein Computerprogrammprodukt, umfassend Befehle, die bei der Ausführung des Programms durch eine Recheneinheit diese veranlassen, ein Verfahren gemäß einer Ausführungsform auszuführen. Das Computerprogrammprodukt kann insbesondere ein computerlesbares Speichermedium aufweisen, umfassend Befehle, die bei der Ausführung des Programms durch eine Recheneinheit diese veranlassen, ein Verfahren gemäß einer Ausführungsform auszuführen.

Ein ophthalmologisches Lasersystem ist dabei ein Lasersystem für die refraktive Augen-Chirurgie. Ein ophthalmologisches Lasersystem kann daher insbesondere dazu ausgelegt sein, mittels des Laserstrahl Schnitte an einer Hornhaut durchzuführen und/oder eine Laserablation von der Hornhaut vorzunehmen. Optional kann das ophthalmologische Lasersystem einen Femtosekunden- und/oder Picosekundenlaser aufweisen.

Eine Strahlführungsbahn ist dabei eine Bahn bzw. ein Verlauf, entlang der bzw. dem der Laserstrahl geführt wird oder geführt werden soll. Die Strahlführungsbahn kann sich dabei senkrecht zur Ausbreitungsrichtung des Laserstrahls erstrecken, also in einer x-y-Ebene. Alternativ oder zusätzlich kann sich die Strahlführungsbahn entlang der Ausbreitungsrichtung des Laserstrahls, also in z-Richtung, erstrecken und etwa eine Änderung der Fokuslage entlang der z-Richtung umfassen. Optional verläuft die Soll-Strahlführungsbahn demnach zumindest teilweise in einer Ebene senkrecht zur Ausbreitungsrichtung des Laserstrahls und/oder zumindest teilweise in einer Richtung parallel zur Ausbreitungsrichtung des Laserstrahls. Eine Soll-Strahlführungsbahn ist dabei eine vorgegebene Strahlführungsbahn, entlang welcher der Laserstrahl geführt werden soll. Eine Ist-Strahlführungsbahn ist dabei eine tatsächliche Strahlführungsbahn, entlang welcher der Laserstrahl tatsächlich geführt wird bzw. verläuft. Zwischen der Soll-Strahlführungsbahn und der zugehörigen Ist-Strahlführungsbahn kann eine Abweichung bestehen, welche typischerweise unerwünscht ist und beispielsweise durch systeminterne Einflüsse und/oder externe Einflüsse verursacht werden kann.

Eine Verstelleinrichtung ist dabei eine Einrichtung, mittels welcher der Laserstrahl abgelenkt und/oder dessen Fokuslage variiert werden kann, um den Laserstrahl entlang einer Strahlführungsbahn zu bewegen. Beispielsweise kann die Verstelleinrichtung einen Linearversteller aufweisen oder als solcher ausgebildet sein, der etwa die Funktion eines z-Scanners zur Anpassung der Fokuslage entlang der z-Richtung innehat. Alternativ oder zusätzlich kann die Verstelleinrichtung zumindest einen x-y-Scanner aufweisen oder als solcher ausgebildet sein.

Ein Bestrahlungsabschnitt ist dabei ein solcher Abschnitt der Strahlführungsbahn, in welchem der Laserstrahl aktiviert ist, d.h. der Laserstrahl auf das zu behandelnde Objekt appliziert wird, um beispielsweise einen Schnitt mit dem Laserstrahl zu erzielen. Dass der Laserstrahl im Bestrahlungsabschnitt eine kontinuierliche Bewegung durchführt bedeutet dabei, dass der Laserstrahl im Bestrahlungsabschnitt nicht zum Stillstand kommt. Es ist nicht zwingend erforderlich, dass der Laserstrahl während des gesamten Bestrahlungsabschnitts dauerhaft aktiviert ist. Optional kann der Laserstrahl während des Bestrahlungsabschnitts auch stellenweise aktiviert und stellenweise deaktiviert sein.

Ein Anpassungsabschnitt ist dabei ein Abschnitt der Strahlführungsbahn, in welchem der Laserstrahl deaktiviert ist und in welchem entsprechend der Laserstrahl nicht auf das zu behandelnde Objekt appliziert wird. Ein Anpassungsabschnitt kann insbesondere dazu dienen, den Laserstrahl für einen beabsichtigten Bestrahlungsabschnitt in Position zu bringen und mit einer vorbestimmten Ablenkgeschwindigkeit und Ablenkrichtung zu versehen, sodass optional keine weitere Anpassung der Position und/oder Ablenk-Geschwindigkeit und/oder Ablenk-Richtung zu Beginn des darauffolgenden Bestrahlungsabschnitts erforderlich ist. Der Anpassungsabschnitt kann dabei insbesondere einen oder mehrere Stillstandspunkte aufweisen, an welchen die Soll-Strahlführungsbahn und/oder die Ist-Strahlführungsbahn derart verlaufen, dass die Verstelleinrichtung zum Stilstand kommt. Sofern im Anpassungsabschnitt mehrere Stillstandspunkte liegen kann das Festlegen der Länge und/oder Zeitdauer des Anpassungsabschnitt und/oder des Abstands des Beginns des Bestrahlungsabschnitts bezüglich des letzten der Stillstandspunkte des Anpassungsabschnitts erfolgen. Ein Stillstandspunkt ist dabei ein Punkt der Ist-Strahlführungskurve und/oder der Soll-Strahlführungskurve, an dem die Verstelleinrichtung für die Positionierung des Laserstrahls zumindest entlang einer Raumrichtung zum Stillstand kommt. Dies schließt nicht aus, dass eine Änderung der Position des Laserstrahls entlang einer oder mehrerer anderer Raumrichtungen erfolgt, wenngleich auch ein Stillstand in einer oder mehrerer der anderen Bewegungsrichtungen zeitgleich vorliegen kann. Sofern sich ein Stillstand über einen längeren Zeitraum erstreckt, stellt das Ende des Zeitraums den Stillstandspunkt dar, zu welchem der Abstand zum Beginn des Bestrahlungsabschnitts festgelegt wird. Der Anpassungsabschnitt kann dabei alternativ oder zusätzlich ein Abschnitt in der Soll-Strahlführungsbahn sein, in welchem auf aktive und/oder passive Weise eine etwaige Abweichung zwischen der Ist-Strahlführungsbahn und der Soll-Strahlführungsbahn verringert wird. Die Verringerung der Abweichung kann dabei derart erfolgen, dass die Abweichung unter einem vorgegebenen Grenzwert liegt. Eine aktive Verringerung der Abweichung kann dabei ein aktives Einwirken auf die Verstelleinrichtung beinhalten, etwa durch das Vorsehen einer Anpassungsbewegung der Verstelleinrichtung in der Soll-Strahlführungsbahn. Eine passive Verringerung der Abweichung kann etwa ein geeignetes Festlegen der Länge bzw. Zeitdauer des Anpassungsabschnitts beinhalten, sodass die Abweichung zwischen der Ist-Strahlführungsbahn und der Soll-Strahlführungsbahn unter einen vorbestimmten Grenzwert fällt, ohne dass eine speziell zur Verringerung der Abweichung ausgestaltete Anpassungsbewegung vorgesehen ist. Dabei kann die Zeitdauer bzw. Länge des Anpassungsabschnitts so gering wie möglich gehalten werden, d.h. als nicht länger festgelegt werden, als für die Verringerung der Abweichung unter den vorbestimmten Grenzwert erforderlich ist.

Die Ausführungsformen bieten den Vorteil, dass durch das Festlegen des Anpassungsabschnitts die Soll-Strahlführungsbahn derart angepasst wird, dass sich die Auswirkungen eines Losbrechsprungs, welche bei Überführen der Verstelleinrichtung aus dem Stillstand in einer kontinuierlichen Bewegung auftreten, bei Eintritt in den Bestrahlungsabschnitt abgeklungen sind. Mit anderen Worten bieten die Ausführungsformen den Vorteil, dass die Soll-Strahlführungsbahn mit einem Anpassungsabschnitt derart versehen wird, dass sich durch den Losbrechsprung ergebende Abweichungen der Ist-Strahlführungsbahn von der Soll-Strahlführungsbahn im Anpassungsabschnitt erschöpfen und sich nicht in den Bestrahlungsabschnitt hinein erstrecken bzw. sich im Bestrahlungsabschnitt unterhalb eines vorbestimmten Grenzwertes befinden.

Die Ausführungsformen bieten somit den Vorteil, dass Abweichungen der Ist-Strahlführungsbahn von der Soll- Strahlführungsbahn während des Bestrahlungsintervalls reduziert oder vermieden werden können und entsprechend die Präzision und Zuverlässigkeit des ophthalmologischen Lasersystems während den Bestrahlungsabschnitten verbessert werden können. Mit anderen Worten ermöglichen die Ausführungsformen, dass etwaige Abweichungen der Ist-Strahlführungsbahn von der Soll-Strahlführungsbahn auf einen Anpassungsabschnitt begrenzt werden können und dadurch deren Auftreten während der Behandlung verhindert werden kann. Dazu kann beispielsweise der Anpassungsabschnitt derart festgelegt werden, dass dieser eine Anpassungsbewegung enthält, während welcher sich die durch den Losbrechsprung hervorgerufenen Abweichungen erschöpfen.

Ferner bieten die Ausführungsformen den Vorteil, dass keine zusätzlichen Anforderungen an das ophthalmologische Lasersystem gestellt werden, insbesondere an der Verstelleinrichtung, sondern vielmehr herkömmliche ophthalmologische Lasersysteme mit herkömmlichen Verstelleinrichtungen verwendet werden können. Insbesondere können ophthalmologische Lasersysteme mit herkömmlichen Verstelleinrichtungen, welche einen Losbrechsprung verursachen, durch die Verwendung eines Verfahrens und/oder einer Steuereinheit gemäß einer Ausführungsform verbessert werden, sodass die Präzision und Zuverlässigkeit ohne kostspielige Hardwareanpassungen verbessert werden können.

Die mit der Überführung der Verstelleinrichtung aus dem Stillstand in die kontinuierliche Bewegung einhergehende Abweichung kann zumindest teilweise durch ein einen Losbrechsprung verursacht werden. Dieser entsteht, wenn die Verstelleinrichtung bei Überführung aus dem Stillstand in einen bewegten Zustand zunächst im Stillstand verharrt, während die Soll-Position sich ändert und die Verstelleinrichtung bei Erreichen einer ausgeprägten Abweichung dann sprunghaft mit einem Losbrechsprung der Soll-Position hinterhereilt. Beispielsweise kann der Losbrechsprung dadurch hervorgerufen werden, dass bei der Überführung der Verstelleinrichtung aus dem Stillstand in den bewegten Zustand zunächst eine Haftreibung überkommen werden muss, wozu eine Losbrechkraft aufgebracht werden muss. Wenn die Verstelleinrichtung sodann in den bewegten Zustand überführt ist, kann die Verstelleinrichtung aufgrund der ausgeübten Losbrechkraft einen als Losbrechmoment bezeichneten mechanischen Impuls innehaben, welcher dazu führt, dass die Verstelleinrichtung mit einem Losbrechsprung der soll-Position hinterhereilt. Dabei stellt das Losbrechmoment einen mechanischen Impuls dar, aber nicht zwingend ein Drehmoment. Vielmehr kann es sich dabei um einen Impuls in Form einer sich geradlinig bewegenden Masse handeln. Die Begriffe Losbrechsprung, Losbrechkraft und Losbrechmoment werden im Folgenden jeweils stets im Zusammenhang mit der der Soll-Strahlführungsbahn nacheilenden Bewegung nach einem Stillstand der Verstelleinrichtung verwendet und bezeichnen das gleiche physikalische Phänomen.

Optional erfolgt das Festlegen einer Länge und/oder Zeitdauer des Anpassungsabschnitts und/oder eines Abstands des Bestrahlungsabschnitts vom (letzten) Stillstandspunkt des Anpassungsabschnitts derart, dass die Länge und/oder Zeitdauer zwischen dem Beginn des Überführens der Verstelleinrichtung aus dem Stillstand in die kontinuierliche Bewegung und dem Beginn der vorgesehenen Bestrahlung des zu behandelnden Objekts gleich oder länger als eine vorbestimmte Sprungzeit und/oder gleich oder länger als ein vorbestimmter Sprungabstand bei der Überführung der Verstelleinrichtung aus dem Stillstand in die kontinuierliche Bewegung ist. Mit anderen Worten wird der Anpassungsabschnitt und/oder ein Abstand des Bestrahlungsabschnitts vom (letzten) Stillstandspunkt des Anpassungsabschnitts unter Berücksichtigung der Sprungzeit und/oder des Sprungabstands festgelegt. Das Festlegen des Sprungabstands kann somit in Abhängigkeit von Eigenschaften der Verstelleinrichtung, insbesondere von Hardwareeigenschaften, erfolgen. Beispielsweise können die Sprungzeit und/oder der Sprungabstand experimentell ermittelt werden und einer geeigneten, systemspezifischen und/oder bauartspezifischen Festlegung des Anpassungsabschnitts und/oder des Abstands des Bestrahlungsabschnitts vom Anpassungsabschnitt zugrunde gelegt werden. Entsprechend kann das Verfahren optional vor dem Festlegen der Länge und/oder Zeitdauer des Anpassungsabschnitts und/oder eines Abstands des Bestrahlungsabschnitts vom (letzten) Stillstandspunkt des Anpassungsabschnitts einen weiteren Schritt aufweisen, welcher ein Ermitteln eins Sprungabstands und/oder einer Sprungzeit der Verstelleinrichtung umfasst. Der Sprungabstand ist dabei jene Wegstrecke, die die Verstelleinrichtung benötigt, bis die vom Losbrechsprung herrührenden Abweichungen unter einen vorbestimmten Grenzwert gefallen sind. Entsprechend ist die Sprungzeit jene Zeitspanne, die die Verstelleinrichtung benötigt, bis die vom Losbrechsprung herrührenden Abweichungen unter einen vorbestimmten Grenzwert gefallen sind. Das Festlegen der Länge und/oder Zeitdauer des Anpassungsabschnitts und/oder eines Abstands des Bestrahlungsabschnitts vom (letzten) Stillstandspunkt des Anpassungsabschnitts derart, dass die Länge und/oder Zeitdauer zwischen dem Beginn des Überführens der Verstelleinrichtung aus dem Stillstand in die kontinuierliche Bewegung und dem Beginn der vorgesehenen Bestrahlung des zu behandelnden Objekts gleich der vorbestimmte Sprungzeit ist, kann dabei eine Minimierung der Zeitdauer bzw. Länge des Anpassungsabschnitts entsprechen. Dies kann den Vorteil bieten, dass die gesamte Behandlungsdauer unter Vermeidung einer Abweichung der Ist-Strahlführungsbahn von der Soll-Strahlführungsbahn bzw. ohne Überschreitung eines vorbestimmten Grenzwertes durch die Abweichung reduziert oder minimiert werden kann.

Optional liegt die vorbestimmte Sprungzeit in einem Bereich von 1 ms bis 200 ms und/oder der vorbestimmte Sprungabstand bei der Überführung der Verstelleinrichtung aus dem Stillstand in die kontinuierliche Bewegung in einem Bereich von 5 µm bis 500 µm. Insbesondere kann bei Verwendung einer als Linearversteller ausgebildeten Verstelleinrichtung die vorbestimmte Sprungzeit und/oder der vorbestimmte Sprungabstand in dem jeweils angegeben Bereich liegen. Bei Verwendung von Galvo-Scannern und/oder Piezo-Scannern können sich etwa kürzere Sprungzeiten und/oder Sprungabstände ergeben.

Optional erfolgt das Festlegen eines Anpassungsabschnitts derart, dass die Soll-Strahlführungsbahn im Anpassungsabschnitt eine Anpassungsbewegung über eine Wegstrecke vorgibt, welche gleich oder länger als der Sprungabstand bei der Überführung der Verstelleinrichtung aus dem Stillstand in die kontinuierliche Bewegung ist. Mit anderen Worten kann im Anpassungsabschnitt eine Bewegung vorgesehen werden, in welcher keine Behandlung des zu behandelnden Objektes stattfindet, sondern welche anstatt dessen eines Abbaus von aus dem Losbrechsprung herrührenden Abweichungen dient. Optional kann im Anpassungsabschnitt auch eine Anpassungsbewegung vorgesehen werden, um aktiv den vom Losbrechsprung herrührenden Abweichungen entgegenzuwirken. Die Anpassungsbewegung kann zudem dazu dienen, die Position und/oder Bewegung der Verstelleinrichtung zum Ende des Anpassungsabschnitts auf eine für den Beginn des darauffolgenden Bestrahlungsabschnitts geeignete Weise bereitzustellen. Die Anpassungsbewegung kann dabei derart gewählt bzw. vorgegeben werden, dass durch die Anpassungsbewegung die Länge und/oder Zeitdauer des Anpassungsabschnitts reduziert oder minimiert wird im Vergleich zu einem Anpassungsabschnitt ohne Anpassungsbewegung (bei passiver Verringerung der Abweichung). Das Vorgeben der Anpassungsbewegung in dem Anpassungsabschnitt kann optional derart erfolgen, dass Abweichungen zwischen einer Ist-Strahlführungsbahn und der Soll-Strahlführungsbahn in kürzerer Zeit abgebaut werden als ohne die Anpassungsbewegung. Dies kann den Vorteil bieten, dass die Behandlungsdauer reduziert oder gar minimiert werden kann.

Optional weist die Anpassungsbewegung eine Bewegung der Verstelleinrichtung mit einer maximalen von der Verstelleinrichtung ausführbaren Beschleunigung auf, und/oder eine sprunghafte Bewegung der Verstelleinrichtung und/oder eine Bewegung entlang eines nicht-differenzierbaren Abschnitts der Soll-Strahlführungsbahn. Dies bietet den Vorteil, dass der Losbrechsprung kontrolliert herbeigeführt werden kann und/oder Abweichungen der Ist-Strahlführungsbahn von der Soll-Strahlführungsbahn zeitlich verkürzt werden können, sodass der Anpassungsabschnitt kurz gehalten werden kann. Dadurch kann die Behandlungsdauer gering gehalten werden. Alternativ oder zusätzlich kann eine Anpassungsbewegung derart vorgegeben werden, dass die Anpassungsbewegung zwischen dem Stillstandspunkt und dem Beginn des darauffolgenden Bestrahlungsabschnitts sowohl eine Beschleunigung als auch ein Verzögern der Verstelleinrichtung entlang zumindest einer Bewegungsrichtung beinhaltet. Mit anderen Worten kann die Anpassungsbewegung optional derart vorgegeben werden, dass der Verlauf der Soll-Strahlführungsbahn zwischen einem Stillstandspunkt und dem Beginn des darauffolgenden Strahlungsabschnitts einen Vorzeichenwechsel der zweiten zeitlichen Ableitung der Position der Verstelleinrichtung aufweist. Dies kann den Vorteil bieten, dass ein zügiger Abbau der Abweichung der Ist-Strahlführungsbahn von der Soll-Strahlführungsbahn erreicht werden kann und entsprechend eine Zeitdauer und/oder Länge des Anpassungsabschnitts reduziert oder minimiert werden kann. Dies kann wiederum den Vorteil bieten, dass die Behandlungsdauer reduziert oder minimiert werden kann. Eine Anpassungsbewegung kann dabei prinzipiell für jede Bewegungsrichtung bzw. Dimension vorgesehen werden, insbesondere für solche Dimensionen, in welchen ein Losbrechsprung der Verstelleinrichtung zu erwarten ist oder auftritt. Dabei können je Dimension ein oder mehrere separate Anpassungsabschnitte festgelegt bzw. vorgesehen sein. Alternativ oder zusätzlich können ein oder mehrere Anpassungsabschnitte festgelegt werden, in welchen jeweils Abweichungen der Ist-Strahlführungsbahn von der Soll-Strahlführungsbahn in mehreren Dimensionen gemeinsam reduziert werden.

Optional kann die Soll-Strahlführungsbahn mehrere Bestrahlungsabschnitte und/oder mehrere Anpassungsabschnitte aufweisen. Beispielsweise kann dadurch eine Strahlführungsbahn für die Erzeugung eines zusammengesetzten Schnittmusters bereitgestellt werden. Insbesondere können dadurch komplexe Schnittmuster erzeugt werden, welche einen mehrmaligen Stillstand der Verstelleinrichtung erfordern. Dazu kann beispielsweise für jeden erforderlichen Stillstand ein separater Anpassungsabschnitt festgelegt werden. Alternativ können mehrere Stillstände in einem Anpassungsabschnitt untergebracht werden. Optional weist die Soll-Strahlführungsbahn zumindest zwei Bestrahlungsabschnitte und zumindest einen Anpassungsabschnitt auf, welcher sich zwischen den beiden Bestrahlungsabschnitten erstreckt.

Optional umfasst der Anpassungsabschnitt und/oder der Bestrahlungsabschnitt ein Überführen der Verstelleinrichtung aus einer kontinuierlichen Bewegung in den Stillstand und das Überführen der Verstelleinrichtung aus dem Stillstand in eine kontinuierliche Bewegung erfolgt im Anpassungsabschnitt zeitlich nach der Überführung der Verstelleinrichtung aus der kontinuierlichen Bewegung in den Stillstand. Mit anderen Worten kann ein Überführen der Verstelleinrichtung aus einer kontinuierlichen Bewegung in den Stillstand im Anpassungsabschnitt oder im Bestrahlungsabschnitt erfolgen. Eine erneute Überführung der Verstelleinrichtung aus dem Stillstand in eine kontinuierliche Bewegung kann sodann in einem anschließenden Anpassungsabschnitt erfolgen. Dies bietet die Möglichkeit, bei Bedarf an einem Stillstand der Verstelleinrichtung flexibel einen Anpassungsabschnitt in die Soll-Strahlführungsbahn einzufügen.

Optional umfasst das Verfahren ferner ein Ermitteln, ob bei einer Überführung der Verstelleinrichtung zur Strahlführung des Laserstrahls aus dem Stillstand an einem bestimmten Stillstandspunkt in eine kontinuierliche Bewegung die damit einhergehende Abweichung der Ist-Strahlführungsbahn von der festgelegten Soll-Strahlführungsbahn den vorbestimmten Grenzwert überschreitet. Danach erfolgt ein Festlegen des Anpassungsabschnitts, sofern ein Überschreiten des vorbestimmten Grenzwerts aufgrund des bestimmten Stillstandspunkts ermittelt wird. Alternativ erfolgt ein Bereitstellen eines Unterbrechungsabschnitts, falls kein Überschreiten des vorbestimmten Grenzwerts aufgrund des bestimmten Stillstandspunkts ermittelt wird, wobei in dem Unterbrechungsabschnitt keine Bestrahlung des zu behandelnden Objektes mit dem Laserstrahl und keine Maßnahme zur Verringerung der mit der Überführung der Verstelleinrichtung aus dem Stillstand in die kontinuierliche Bewegung einhergehenden Abweichung der Ist-Strahlführungsbahn von der festgelegten Soll-Strahlführungsbahn vorgesehen ist. Dies bietet die Möglichkeit, einen Anpassungsabschnitt nur dann vorzusehen, wenn dieser auch benötigt wird, um ein Überschreiten des vorbestimmten Grenzwerts durch die aus dem Losbrechsprung herrührenden Abweichungen zu vermeiden. Sofern ohnehin ein Überschreiten des Grenzwertes nicht zu erwarten ist, kann hingegen auf einen Anpassungsabschnitt verzichtet werden und lediglich ein Unterbrechungsabschnitt eingefügt werden. Dadurch kann die Behandlungsdauer gering gehalten werden. Somit kann die Soll-Strahlführungsbahn einen oder mehrere Anpassungsabschnitte und/oder einen oder mehrere Unterbrechungsabschnitte aufweisen.

Der vorbestimmte Grenzwert kann in Abhängigkeit eines Positionsfehlers bestimmt sein, bei welchem bei Auftreten während eines Bestrahlungsabschnitts ein Abbruch der Bestrahlung des zu behandelnden Objektes mittels des Laserstrahls erfolgen würde bzw. zu erfolgen hat. Beispielsweise kann der vorbestimmte Grenzwert derart festgelegt werden, dass dieser einem Anteil von 25%, oder 50% oder 75% des maximal erlaubten Positionsfehlers entspricht, bei welchem ein Abbruch der Behandlung erfolgen würde. Dadurch können unerwünschte Unterbrechungen und/oder Abbrüche der Behandlung aufgrund des Losbrechsprungs vermieden werden und dennoch die Behandlungsdauer kurz gehalten werden.

Optional ist die Steuereinheit ferner dazu eingerichtet, die Verstelleinrichtung zu steuern und/oder zu regeln. Mit anderen Worten kann die Steuereinheit optional zusätzlich zur Funktion, gemäß welcher sie die Soll-Strahlführungsbahn bereitstellt und ggf. als Planungseinheit dient, auch die Funktion zur Steuerung und/oder Regelung der Verstelleinrichtung aufweisen. Beispielsweise kann die Steuereinheit als Computer ausgebildet sein und mehrere Funktionen innehaben.

Optional umfasst das ophthalmologische Lasersystem ferner eine Laserstrahlaktivierungseinheit, mittels welcher der Laserstrahl aktivierbar und deaktivierbar ist, wobei die Laserstrahlaktivierungseinheit optional zumindest eines der folgenden Elemente aufweist: einen akustooptischen Modulator (AOM), einen Shutter. Dadurch kann bei Bedarf der Laserstrahl auf einfache Weise aktiviert und deaktiviert werden, um den Laserstrahl auf das zu behandelnde Objekt zu applizieren oder dies zu unterbinden. Eine Laserstrahldeaktivierungseinheit kann den Vorteil bieten, dass der Laserstrahl deaktiviert werden kann, indem er beispielsweise blockiert oder auf geeignete Weise umgelenkt wird. Dadurch kann ein Erfordernis entfallen, die Laserquelle kurzzeitig abzuschalten, sodass ein kontinuierlicher und stabiler Betrieb der Laserquelle aufrechterhalten werden kann. Ein Computerprogrammprodukt kann beispielsweise in Form eines Programmcodes vorliegen, welcher auf einem physikalischen Datenträger gespeichert ist und/oder über ein Netzwerk heruntergeladen werden kann. Ein computerlesbares Speichermedium kann demnach in Form eines physikalischen Datenträgers oder einer Speichereinheit vorliegen, auf welcher ein entsprechender Programmcode gespeichert ist.

Die oben genannten und im Folgenden erläuterten Merkmale und Ausführungsformen sind dabei nicht nur als in den jeweils explizit genannten Kombinationen offenbart anzusehen, sondern sind auch in anderen technisch sinnhaften Kombinationen und Ausführungsformen vom Offenbarungsgehalt umfasst. Die auf das Verfahren gerichtete Offenbarung ist auch als für die Steuereinheit und das ophthalmologische Lasersystem offenbart anzusehen und umgekehrt.

Die Erfindung wird durch die angehängten Ansprüche definiert.

Weitere Einzelheiten und Vorteile sollen nun anhand von den folgenden Beispielen und optionalen Ausführungsformen mit Bezug auf die Figuren näher erläutert werden.

Es zeigen:
- Figur 1:: in einer schematischen Darstellung ein ophthalmologisches Lasersystem 10 gemäß einer optionalen Ausführungsform;
- Figur 2A:: beispielhaft in Diagrammen eine Gegenüberstellung von Soll-Strahlführungsbahnen eines herkömmlichen ophthalmologischen Lasersystems (Fig. 2B) und eines ophthalmologischen Lasersystems gemäß einer optionalen Ausführungsform (Fig. 2B);
- Figur 3:: den zeitlichen Verlauf einer weiteren beispielhaften Strahlführungsbahn eines ophthalmologischen Lasersystems gemäß einer optionalen Ausführungsform.
- Figur 4:: eine beispielhafte Erläuterung des Losbrechsprungs.
- Figuren 5 bis 8:: optionale Ausführungsformen von Verfahren zur Vermeidung und/oder Kompensation von Abweichungen aufgrund des Losbrechsprungs.

In den folgenden Figuren werden gleiche oder ähnliche Elemente in den verschiedenen Ausführungsformen der Einfachheit halber mit gleichen Bezugszeichen bezeichnet.

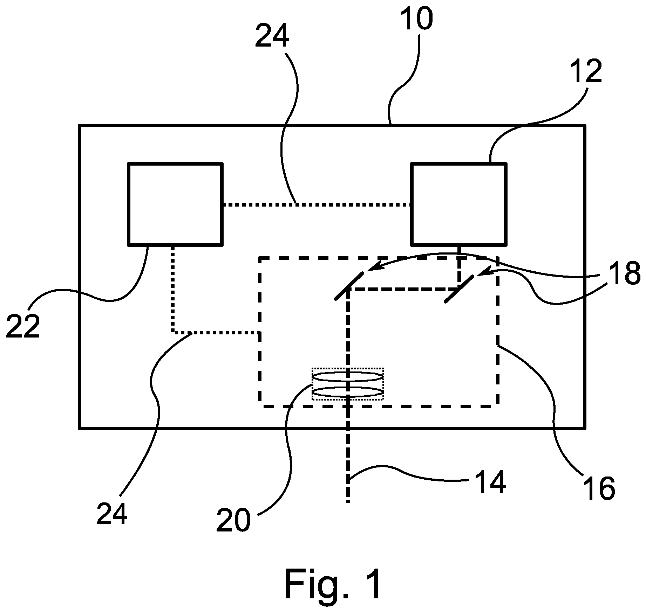

Figur 1 zeigt in einer schematischen Darstellung ein ophthalmologisches Lasersystem 10 gemäß einer optionalen Ausführungsform. Das ophthalmologische Lasersystem 10 weist eine Laserquelle 12 auf, welche dazu ausgelegt ist, einen für die Behandlung eines Objektes, insbesondere eines Patientenauges, geeigneten Laserstrahl 14 zu emittieren. Der Laserstrahl 14 kann dabei als eine Dauerstrichlaserstrahlung oder als gepulste Laserstrahlung vorliegen, wobei die Laserpulse optional als Pikosekunden- oder als Femtosekundenlaserpulse ausgebildet sein können. Das ophthalmologische Lasersystem 10 weist ferner eine Verstelleinrichtung 16 auf, mittels welcher der Laserstrahl 14 abgelenkt werden kann und gemäß einer Strahlführungsbahn räumlich variiert werden kann, um das zu behandelnde Objekt entlang der Strahlführungsbahn mit dem Laserstrahl zu beaufschlagen. Gemäß der gezeigten Ausführungsform weist die Verstelleinrichtung 16 einen x-y-Scanner 18 auf, welcher zwei steuerbare Spiegel aufweist, um den Laserstrahl in einer Ebene senkrecht zur Ausbreitungsrichtung des Laserstrahls 14 bewegen zu können. Zudem weist die Verstelleinrichtung einen z-Scanner 20 auf, mittels welchem die Fokuslage in einer Richtung parallel zur Ausbreitungsrichtung des Laserstrahls 14 variiert bzw. bewegt werden kann. Beispielsweise kann der z-Scanner eine optische Linsenanordnung aufweisen, welche mittels eines Linearverstellers, etwa durch einen Schrittmotor angetrieben, präzise positioniert werden kann. Die Verstelleinrichtung ist dabei dadurch gekennzeichnet, dass diese beim Überführen aus dem Stillstand in einer kontinuierlichen Bewegung einen Losbrechsprung hervorruft. D.h. dass beim Anfahren der Verstelleinrichtung zunächst eine verzögerte und ruckartige Bewegung der Verstelleinrichtung auftritt, welche zur Folge hat, dass die tatsächliche Positionierung der Verstelleinrichtung zunächst von der beabsichtigten Positionierung abweicht. Gemäß anderen optionalen Ausführungsformen kann die Verstelleinrichtung jedoch auch nur einen x-y-Scanner oder nur einen z-Scanner aufweisen.

Außerdem weist das ophthalmologische Lasersystem 10 eine Steuereinheit 22 auf, welche dazu eingerichtet ist, eine Soll-Strahlführungsbahn zur Strahlführung des Laserstrahls bereitzustellen. Die Steuereinheit ist dabei dazu ausgelegt, die Soll-Strahlführungsbahn für die Steuerung der Verstelleinrichtung 16 bereitzustellen. Dazu kann beispielsweise die Steuereinheit eine Kommunikationsverbindung 24 aufweisen, über welche die Steuereinheit 22 sodann die Daten zur Soll-Strahlführungsbahn an die Verstelleinrichtung 16 übermitteln kann, oder über welche die Steuereinheit 22 die Verstelleinrichtung 16 gemäß der Soll-Strahlführungsbahn direkt steuern kann.

Zur Bereitstellung der Soll-Strahlführungsbahn ist die Steuereinheit 22 dazu eingerichtet, einen Anpassungsabschnitt und einen Bestrahlungsabschnitt festzulegen, welche jeweils einen Teil der Soll-Strahlführungsbahn bilden. Ebenso kann die Steuereinheit 22 dazu eingerichtet sein, eine Sollstrahlführungsbahn mit mehreren Anpassungsabschnitten und/oder mehreren Bestrahlungsabschnitten bereitzustellen.

Ein Anpassungsabschnitt wird insbesondere dann von der Steuereinheit 22 vorgesehen, wenn in einem Abschnitt die Verstelleinrichtung 16 zur Strahlführung des Laserstrahls 14 aus dem Stillstand in eine kontinuierliche Bewegung zu überführen ist und ein Losbrechsprung zu erwarten ist, welche nach dem Überführen der Verstelleinrichtung 16 in eine kontinuierliche Bewegung zur einer Abweichung der Soll-Strahlführungsbahn von der Ist-Strahlführungsbahn führt. Dabei kann optional zunächst ermittelt werden, ob ein Losbrechsprung zu erwarten ist und, falls dem so ist, ob die damit einhergehende Abweichung einen vorbestimmten Grenzwert überschreitet oder nicht. Sofern eine Überschreitung des Grenzwerts zu erwarten ist, kann ein Anpassungsabschnitt obsolet sein und entsprechend kann auf eine Anpassung der Länge und/oder Zeitdauer des Anpassungsabschnitts verzichtet werden. Vielmehr kann etwa das Einfügen eines Unterbrechungsabschnitts in die Soll-Strahlführungsbahn ohne einem Entgegenwirken gegen die Abweichung genügen. Ist hingegen eine größere Abweichung als der vorbestimmte Grenzwert zu erwarten, kann die Steuereinheit 22 einen entsprechend ausgestalteten Anpassungsabschnitt bestimmen und diesen in die Soll-Strahlführungsbahn implementieren.

Ein Anpassungsabschnitt kann insbesondere vor einem Bestrahlungsabschnitt und/oder zwischen zwei Bestrahlungsabschnitten vorgesehen werden, in welchen der Laserstrahl 14 positioniert und (zumindest entlang einer Bewegungsrichtung) zum Stilstand gebracht werden muss, um sodann den Bestrahlungsabschnitt wie vorgesehen durchführen bzw. durchfahren zu können, sofern mit dem jeweiligen Stillstand ein Losbrechsprung mit einer den Grenzwert übersteigenden Abweichung verbunden ist. Da während des Anpassungsabschnitts keine Bestrahlung des zu behandelnden Objektes erfolgt, kann der Laserstrahl 14 frei bewegt werden, ohne dass dies Auswirkungen auf das zu behandelnde Objekt hat. Der Bestrahlungsabschnitt der Soll-Strahlführungsbahn wird dabei von der Steuereinheit 22 derart festgelegt, dass dieser an den bzw. an einen der mehreren Anpassungsabschnitte angrenzt und dass in dem Bestrahlungsabschnitt mittels der Verstelleinrichtung 16 der Laserstrahl 14 eine kontinuierliche Bewegung durchführt und eine Bestrahlung des zu behandelnden Objektes mit dem Laserstrahl 14 erfolgt.

Konkret ist das ophthalmologisches Lasersystem 10 dabei dazu eingerichtet ist, eine mit dem Losbrechsprung einhergehende Abweichung der Ist-Strahlführungsbahn von der Soll-Strahlführungsbahn, die größer als der vorbestimmte Grenzwert ist, zu ermitteln und eine Länge und/oder Zeitdauer des Anpassungsabschnitts und/oder eines Abstands des Beginns des Bestrahlungsabschnitts vom Stillstandspunkt des Anpassungsabschnitts derart festzulegen, dass die Abweichung der Ist-Strahlführungsbahn von der Soll-Strahlführungsbahn im gesamten Bestrahlungsabschnitt kleiner als ein vorbestimmter Grenzwert ist. Mit anderen Worten sollen jene Bereiche der Ist-Strahlführungsbahn, in welchen die Abweichung der Ist-Strahlführungsbahn von der Soll-Strahlführungsbahn größer als der vorbestimmte Grenzwert ist, gänzlich in einem Anpassungsabschnitt liegen und sich nicht in einen Bestrahlungsabschnitt erstrecken. Beispielsweise können dazu Informationen über die der Verstelleinrichtung anhaftende Losbrechkraft und den dadurch verursachten Losbrechsprung in der Steuereinheit 22 hinterlegt sein, auf Basis welcher die Steuereinheit 22 sodann die Zeitdauer und/oder die Wegstreckenlänge des Anpassungsabschnitts entsprechend festlegen kann, sodass die daraus resultierenden Abweichungen zwischen Soll-Strahlführungsbahn und Ist-Strahlführungsbahn innerhalb des Anpassungsabschnitts abgeklungen und unter einen vorgegebenen Grenzwert gesunken sind.

Zur Ermittlung der Ist-Strahlführungsbahn, anhand welcher die Steuereinheit 22 eine etwaige Abweichung der Ist-Strahlführungsbahn von der Soll-Strahlführungsbahn bestimmen kann, kann das ophthalmologische Lasersystem 10 beispielsweise eine Überwachungseinheit aufweisen (nicht gezeigt). Die Überwachungseinheit kann etwa eine Kamera und/oder einen anderweitigen optischen Sensor aufweisen, mittels welchem ein Bild vom zu behandelnden Objekt, etwa der Hornhaut eines zu behandelnden Patientenauges, und dem darauf einfallenden Laserstrahl 14 erfasst und von der Steuereinheit ausgewertet werden kann. Alternativ oder zusätzlich kann die Überwachungseinheit ein Längenmesssystem aufweisen, etwa ein Linearpotentiometer an einer als Linearversteller ausgebildeten Verstelleinrichtung, um die Abweichung zu bestimmen. Dies kann eine zuverlässige Ermittlung der Abweichung ermöglichen.

Eine derartige Festlegung der Soll-Strahlführungsbahn durch die Steuereinheit 22 bietet den Vorteil, dass die durch einen Losbrechsprung der Verstelleinrichtung 16 verursachten Abweichungen der Ist-Strahlführungsbahn von der Soll-Strahlführungsbahn bereits in einem Anpassungsabschnitt, in welchem keine Bestrahlung des zu behandelnden Objekts erfolgt, unter einen vorgegebenen Grenzwert reduziert werden und entsprechend in einem darauffolgenden Bestrahlungsabschnitt keine Abweichungen, die den vorgegebenen Grenzwert überschreiten, aufgrund des Losbrechsprungs auftreten. Somit kann eine besonders hohe Präzision der Bestrahlung und damit vorgenommenen Schritten erzielt werden, ohne die Ablenkgeschwindigkeit im Bestrahlungsabschnitt und bei der Repositionierung des Laserstrahls erheblich reduzieren zu müssen.

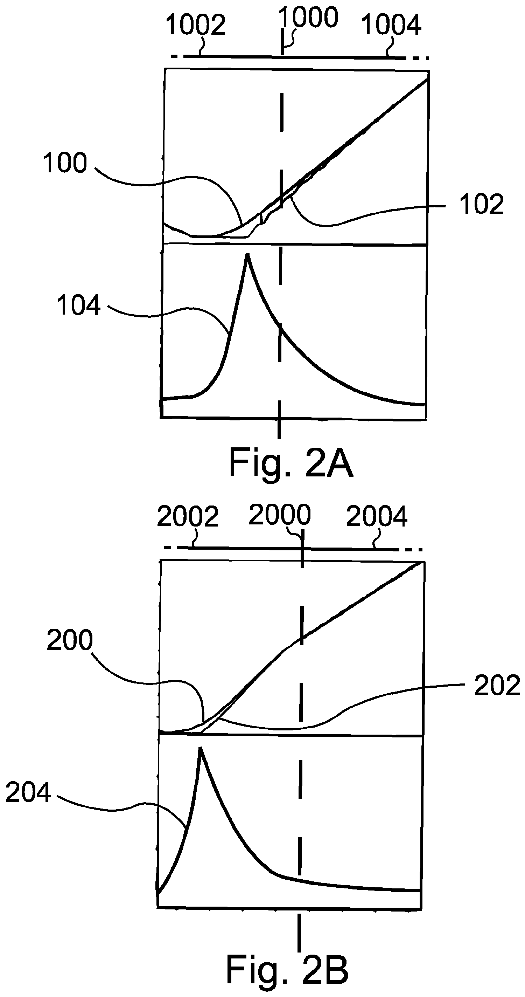

Die Figuren 2A und 2B zeigen beispielhaft in Diagrammen eine Gegenüberstellung von Soll-Strahlführungsbahnen eines herkömmlichen ophthalmologischen Lasersystems (Fig. 2A) und eines ophthalmologischen Lasersystems 10 gemäß einer optionalen Ausführungsform (Fig. 2B).

Dabei sind jeweils im oberen Graphen die vorgegebene Soll-Strahlführungsbahn 100, 200 und die tatsächliche Ist-Strahlführungsbahn 102, 202 als Auslenkung in beliebigen Einheiten gegen die Zeit aufgetragen. Im unteren Abschnitt ist die Differenz zwischen der Ist-Strahlführungsbahn und der Soll-Strahlführungsbahn gegen die Zeit aufgetragen. Die gestrichelte Linie 1000 bzw. 2000 markiert dabei jeweils die Grenze zwischen dem Anpassungsabschnitt 1002, 2002, welcher linkerhand für bis zur Grenze 1000, 2000 reicht und einem darauffolgenden Bestrahlungsabschnitt 1004, 2004, welcher bei der Grenze 1000, 2000 beginnt und sich ab dieser mit fortschreitender Zeit erstreckt.

Der in Figur 2A dargestellte Fall eines herkömmlichen ophthalmologischen Lasersystems zeigt, dass im Anpassungsabschnitt 1002 zeitweise die Position bzw. Auslenkung der Soll-Strahlführungsbahn100 und Ist-Strahlführungsbahn 102 unverändert auf einem Wert verharrt. In diesem Zeitraum befindet sich die Verstelleinrichtung im Stillstand. Im weiteren Verlauf des Anpassungsabschnitts 1002 steigt die Auslenkung der Soll-Strahlführungsbahn 100 wieder an und steigert sich kontinuierlich, was eine kontinuierliche Bewegung der Verstelleinrichtung 16 bewirken soll. Allerdings ist zu erkennen, dass die Ist-Strahlführungsbahn 102 der Soll-Strahlführungsbahn 100 hinterherhinkt und die Verstelleinrichtung 16 länger im Stillstand verharrt, als dies durch die Soll-Strahlführungsbahn 100 vorgegeben ist. Dadurch entsteht eine deutliche Diskrepanz bzw. Differenz zwischen der Ist-Strahlführungsbahn 102 und der Soll-Strahlführungsbahn 100, deren zeitlicher Verlauf in Graph 104 im unteren Graphen dargestellt ist. Erst wenn die Differenz bzw. Abweichung bereits deutlich angewachsen ist, löst sich die Verstelleinrichtung 16 aus dem Stillstand und wird in einem Losbrechsprung ruckartig aus dem Stillstand in Bewegung versetzt, wodurch die Verstelleinrichtung 16 eine Bewegung mit einem deutlich ausgeprägten Losbrechsprung ausführt und der Soll-Strahlführungsbahn 100 nacheilt. Nachdem sich die Verstelleinrichtung des herkömmlichen ophthalmologischen Lasersystems in Bewegung versetzt hat, verringert diese sukzessive die Differenz zwischen der Ist-Strahlführungsbahn 102 und der Soll-Strahlführungsbahn 100 unter Ausführung einer Bewegung, welche durch den Losbrechsprung hervorgerufen wird. Dabei ist deutlich zu erkennen, dass auch nach Überschreiten der Grenze 1000, also während des Bestrahlungsabschnitts 1004 die Differenz bzw. Abweichung zwischen der Ist-Strahlführungsbahn 100 und der Soll-Strahlführungsbahn 102 noch nicht vollständig abgeklungen ist und entsprechend zu einer Abweichung von der vorgegebenen Soll-Strahlführungsbahn führt. Die Zeitdauer des Auftretens der Differenz zwischen der Ist-Strahlführungskurve 102 und der Soll-Strahlführungskurve 100 bis zu ihrer Reduktion unter den vorbestimmten Grenzwert entspricht dabei der Sprungzeit. Dadurch wird die Präzision der Bestrahlung und von mittels der Bestrahlung erzeugten Schnitte reduziert, was zu fehlerhaften Behandlungen oder zu einem durch eine etwaige Sicherheitsvorrichtung herbeigeführten Behandlungsabbruch führen kann. In herkömmlichen Systemen könnte die Abweichung dadurch reduziert werden, dass die Ablenkgeschwindigkeit reduziert wird. Dies geht jedoch mit einer entsprechenden Verlangsamung der Behandlung und einer längeren Behandlungsdauer einher.

In Figur 2B ist das gleiche Szenario für ein ophthalmologisches Lasersystem 10 gemäß einer optionalen Ausführungsform illustriert. Im Vergleich zum herkömmlichen ophthalmologischen Lasersystem (Figur 2A) berücksichtig dieses bei der Bereitstellung der Soll-Strahlführungsbahn 200 ein bei der Überführung der Verstelleinrichtung 16 auftretender Losbrechsprung, indem die Dauer bzw. Länge des Anpassungsabschnitts entsprechend angepasst wird. Wie in Figur 2B zu erkennen ist, tritt auch bei dem ophthalmologischen Lasersystem 10 gemäß der optionalen Ausführungsform aufgrund des Losbrechsprungs eine Differenz bzw. Abweichung zwischen der Ist-Strahlführungsbahn 200 und der Soll-Strahlführungsbahn 202 auf, da die Verstelleinrichtung 16 länger im Stillstand verharrt, als durch die Soll-Strahlführungsbahn 200 vorgegeben. Allerdings ist die Dauer des Anpassungsabschnitts 2002 derart lange gewählt, dass die Differenz zwischen der Ist-Strahlführungsbahn 200 und der Soll-Strahlführungsbahn 202, wie in Graph 204 gezeigt, bereits innerhalb des Anpassungsabschnitts 2002 nahezu vollständig abklingt und nach der Grenze 2004 im Bestrahlungsabschnitt 2004 unterhalb eines vorbestimmten Grenzwertes liegt. Entsprechend kommt es im Bestrahlungsabschnitt 2004 zu keiner signifikanten Differenz zwischen der Ist-Strahlführungsbahn 200 und der Soll-Strahlführungsbahn 202. Entsprechend kann mit dem ophthalmologischen Lasersystem 10 gemäß der optionalen Ausführungsform die Bestrahlung bzw. Behandlung mit einer deutlich höheren Präzision durchgeführt werden, als dies mit einem herkömmlichen ophthalmologischen Lasersystem möglich ist, wie in Figur 2A geschildert.

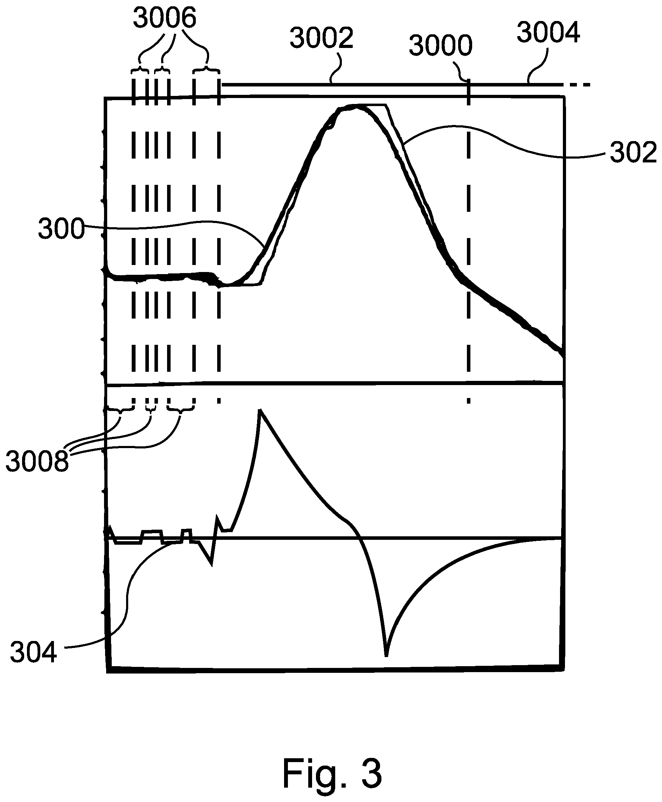

Figur 3 zeigt den zeitlichen Verlauf einer weiteren beispielhaften Strahlführungsbahn eines ophthalmologischen Lasersystems 10 gemäß einer optionalen Ausführungsform. Diese weist einen Anpassungsabschnitt 3002 auf, an welchen sich an der Grenze 3000 ein Bestrahlungsabschnitt 3004 anschließt. Außerdem weist die Strahlführungsbahn weitere kleinere Bestrahlungsabschnitte 3006 mit dazwischen liegenden Unterbrechungsabschnitten 3008 auf. Auch gemäß dieser optionalen Ausführungsform ist die Länge bzw. Dauer des Anpassungsabschnitts derart gewählt, dass sich eine Abweichung bzw. Differenz der Ist-Strahlführungsbahn 302 von der Soll-Strahlführungsbahn 300 aufgrund des bei Übergang der Verstelleinrichtung vom Stillstand am Stillstandspunkt, welcher sich am Scheitelpunkt der Soll-Strahlführungsbahn befindet, in eine kontinuierliche Bewegung auftretenden Losbrechsprungs innerhalb des Anpassungsabschnitts 3002 unterhalb einen vorgegebenen Grenzwert reduziert. Entsprechend wird auch gemäß dieser Ausführungsform im Bestrahlungsabschnitt 3004 eine sehr geringe und nahezu verschwindende Differenz 304 der Ist-Strahlführungsbahn 302 von der Soll-Strahlführungsbahn 300 erzielt. Der Anpassungsabschnitt 3002 dient dabei dazu, eine Verlaufsrichtung der Ist-Strahlführungsbahn 302 zu realisieren, welche zwischen dem letzten kleinen Bestrahlungsabschnitt 3006 und dem Bestrahlungsabschnitt 3004 einen Knick aufweist, welcher zu einem nicht-stetigdifferenzierbaren Übergang in der Soll-Strahlführungsbahn 300 führen würde, wenn die Bestrahlungsabschnitte 3006 und 3004 ohne Unterbrechung aneinander gereiht werden würden. Ein solcher Knick würde demnach eine unendliche Beschleunigung in der Soll-Strahlführungsbahn erfordern. Um dies zu vermeiden und auch eine Abweichung der Ist-Strahlführungsbahn von der Soll-Strahlführungsbahn im Bestrahlungsabschnitt 3004 zu verhindern, ist der Anpassungsabschnitt 3002 eingefügt, mittels welchem beides erreicht werden kann. Dabei tritt nach dem Scheitpunkt der Soll-strahlführungsbahn 300, an welchem der Stillstandspunkt liegt, ein Losbrechsprung auf, welcher eine Abweichung zwischen der Ist-Strahlführungsbahn 302 und der Soll-Strahlführungsbahn 300 verursacht. Dabei erstreckt sich der Anpassungsabschnitt 3002 vom Stillstandspunkt derart weit bzw. lange, dass die Abweichung bis zum Beginn des anschließenden Bestrahlungsabschnitts 3004 abgebaut ist oder zumindest einen vorbestimmten Grenzwert nicht mehr übersteigt.

Die gezeigte Ausführungsform weist zudem die weiteren Bestrahlungsabschnitte 3006 auf, welche sich zeitlich vor dem Anpassungsabschnitt 3002 erstrecken. Zwischen diesen Bestrahlungsabschnitten 3006 weist die Strahlführungsbahn jeweils einen Unterbrechungsabschnitt 3008 auf, in welchem keine Bestrahlung des zu behandelnden Objekts mit dem Laserstrahl stattfindet. Diese Unterbrechungsabschnitte 3008 können beispielsweise dazu dienen, den Laserstrahl 14 bzw. die Verstelleinrichtung 16 zu repositionieren, wobei in bei diesen Repositionierungen kein Losbrechsprung auftritt oder eine damit einhergehende Abweichung derart gering ist, dass diese den vorbestimmten Grenzwert nicht überschreitet und entsprechend nicht kompensiert werden muss. Dies kann etwa dann der Fall sein, wenn die Bewegung der Soll-Strahlführungsbahn sehr klein ist und entsprechend keine signifikanten Abweichungen auftreten können, da die Bewegung selbst kleiner als der vorbestimmte Grenzwert ist. In solchen Fällen kann demnach bewusst auf eine Kompensation einer Abweichung verzichtet werden und entsprechend ein Unterbrechungsabschnitt 3008 anstatt eines Anpassungsabschnitts 3002 eingefügt werden. Beispielsweise können solche Unterbrechungsabschnitte 3008 genutzt werden, um Unterbrechungszeiten zu schaffen, in welchen andere Verstelleinrichtungen (etwa für andere Dimensionen) und/oder die Laserquelle verstellt werden.

Der vorbestimmte Grenzwert kann etwa anhand des maximal erlaubten Positionsfehlers bestimmt sein, ab welchem ein Abbruch der Behandlung erforderlich ist. Beispielsweise kann der vorbestimmte Grenzwert derart vorbestimmt sein, dass dieser 25%, 50% oder 75% des maximal erlaubten Positionsfehlers beträgt. Wird somit eine Abweichung der Ist-Strahlführungsbahn von der Soll-Strahlführungsbahn festgestellt oder erwartet, die den vorbestimmten Grenzwert überschreitet, kann ein Anpassungsabschnitt 3002 vorgesehen werden. Wird hingegen eine Abweichung festgestellt oder erwartet, die den vorbestimmten Grenzwert nicht überschreitet, kann es sinnvoll sein, keine Kompensation eines Losbrechsprungs vorzunehmen und entsprechend einen Unterbrechungsabschnitt 3008 anstatt eines Anpassungsabschnitts 3002 vorzusehen.

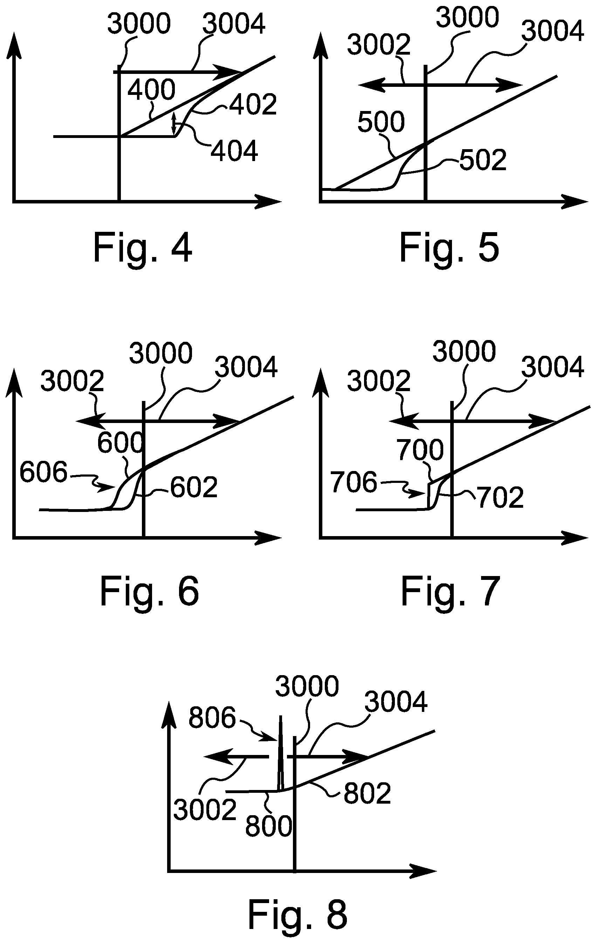

Gemäß optionalen Ausführungsformen kann eine durch einen Losbrechsprung verursachte Abweichung nicht nur durch eine Länge und/oder Zeitdauer des Anpassungsabschnitts 3002 vom Bestrahlungsabschnitt 3004 ferngehalten werden, sondern es kann alternativ oder zusätzlich eine Anpassungsbewegung im Anpassungsabschnitt 3002 bereitgestellt werden, um den Losbrechsprung zumindest teilweise zu kompensieren. Dazu sind in Figur 4 das zugrundeliegende Problem und in den Figuren 5 bis 8 verschiedene Formen des Anpassungsabschnitts und optional einer Anpassungsbewegung beispielhaft erläutert, wobei jeweils auf der horizontalen Achse die Zeit und auf der vertikalen Achse die Auslenkung der Verstelleinrichtung 16, jeweils in beliebigen Einheiten, angegeben ist. In allen der im Folgenden geschilderten Fälle ist die Verstelleinrichtung 16 vor dem Bestrahlungsabschnitt 3004 im Stillstand und wird in eine kontinuierliche Bewegung überführt, wobei ein Losbrechsprung auftritt.

Figur 4 zeigt ein Szenario, bei welchem in einem Bestrahlungsabschnitt 3004 eine lineare Bewegung der Verstelleinrichtung 16 erfolgen soll und vor dem Bestrahlungsabschnitt 3004 die Verstelleinrichtung 16 im Stillstand verweilt. Die Soll-Strahlführungsbahn 400 beginnt entsprechend an der Grenze 3000 linear zu steigen. Aufgrund der Haftreibung verweilt jedoch die Ist-Strahlführungsbahn 402 zunächst weiterhin im Stillstand, bis die Abweichung zwischen Ist-Strahlführungsbahn 402 und Soll-Strahlführungsbahn 400 derart ausgeprägt ist, dass mittels der Losbrechkraft die Haftreibung überwunden werden kann und die Verstelleinrichtung 16 in einem Losbrechsprung der Soll-Strahlführungsbahn 400 nacheilt und auf diese Weise die Abweichung der Ist-Strahlführungsbahn 402 von der Soll-Strahlführungsbahn 400 reduziert. Dabei ist erkennbar, dass ohne eine Kompensation des Losbrechsprungs eine deutliche Abweichung 404 der Ist-Strahlführungsbahn 402 von der Soll-Strahlführungsbahn 400 während des Behandlungsabschnitts 3004 auftritt, was zu einem ungewollten Abbruch der Behandlung führen kann.

Gemäß Figur 5 wird ein Anpassungsabschnitt 3002 bereitgestellt, in welchem die Verstelleinrichtung 16 aus dem Stillstand in eine kontinuierliche Bewegung versetzt wird, wobei sich der Anpassungsabschnitt 3002 nach dem Stillstand der Verstelleinrichtung 16 derart lange erstreckt, dass die durch den Losbrechsprung auftretenden Abweichungen der Ist- Strahlführungsbahn 502 von der Soll-Strahlführungsbahn 500 nicht oder nur unwesentlich in den sich anschließenden Bestrahlungsabschnitt 3004 hinein erstrecken. Dass sich die Abweichungen unwesentlich in den Bestrahlungsabschnitt 3004 hinein erstrecken bedeutet dabei, dass im Bestrahlungsabschnitt 3004 die Abweichungen derart klein sind, dass diese einen vorbestimmten Grenzwert nicht überschreiten.

Gemäß der optionalen Ausführungsform in Figur 6 findet der Losbrechsprung in einem dafür vorgesehenen Anpassungsabschnitt 3002 statt, wie auch in der in Figur 5 geschilderten Ausführungsform. Abweichend davon sieht jedoch in dieser Ausführungsform die Soll-Strahlführungsbahn 600 eine Anpassungsbewegung 606 vor, bei welcher die Verstelleinrichtung 16 mit maximale Geschwindigkeit in die gewünschte Richtung bewegt wird, um das Auftreten des Losbrechsprungs kontrolliert und mit möglichst geringer Zeitdauer auftreten zu lassen. Dies bietet den Vorteil, dass der Anpassungsabschnitt zeitlich reduziert werden kann, da die Abweichungen zwischen der Ist-Strahlführungsbahn 602 und der Soll-Strahlführungsbahn 600 in kürzerer Zeit abgebaut werden können. Gemäß dieser Ausführungsform erfolgt die Anpassungsbewegung derart, dass zwischen dem Stillstandspunkt und der Grenze 3000 zum nächsten Bestrahlungsabschnitt 3004 sowohl eine Beschleunigung als auch eine Verzögerung der Verstelleinrichtung erfolgt. Mit anderen Worten beinhaltet der Bereich zwischen dem Stillstandspunkt und der Grenze 3000 zum nächsten Bestrahlungsabschnitt 3004 einen Vorzeichenwechsel der zweiten zeitlichen Ableitung der Position der Verstelleinrichtung.

Auch in der in Figur 7 geschilderten Ausführungsform wird, ähnlich wie mit Bezug auf Figur 6 erläutert, eine Anpassungsbewegung 706 in der Soll-Strahlführungsbahn 700 vorgesehen, um den Losbrechsprung in der Ist-Strahlführungsbahn 702 kontrolliert und zeitlich verkürzt hervorzurufen. Abweichend von Figur 6 wird gemäß Figur 7 dazu ein Sprung in der Soll-Strahlführungsbahn 700 bereitgestellt.

Gemäß Figur 8 wird eine Anpassungsbewegung 806 in Form einer Spitze in der Soll-Strahlführungsbahn 800 vorgesehen. Das Einführen der Spitze in der Soll-Strahlführungsbahn 800 dient dazu, ein nahezu instantanes Losbrechen der Verstelleinrichtung 16 im Anpassungsabschnitt 3002 der Ist-Strahlführungsbahn 802 hervorzurufen. Der Anpassungsabschnitt 3002 kann auch durch diese Anpassungsbewegung 806 zeitlich kurz gehalten werden.

Es wird darauf hingewiesen, dass auch andere Formen der Anpassungsbewegung geeignet sein können, um den Losbrechsprung gering zu halten und/oder die dadurch entstehenden Abweichungen der Ist-Strahlführungsbahn von der Soll-Strahlführungsbahn zu minimieren und/oder zeitlich möglichst kurz zu halten.

### Bezugszeichenliste

- 10: ophthalmologisches Lasersystem
- 12: Laserquelle
- 14: Laserstrahl
- 16: Verstelleinrichtung
- 18: x-y-Scanner
- 20: z-Scanner
- 22: Steuereinheit
- 24: Kommunikationsverbindung

- 100: Soll-Strahlführungsbahn eines herkömmlichen Lasersystems
- 102: Ist-Strahlführungsbahn eines herkömmlichen Lasersystems
- 104: Differenz der Ist-Strahlführungsbahn und Soll-Strahlführungsbahn eines herkömmlichen Lasersystems

- 1000: Grenze zwischen herkömmlichem Anpassungsabschnitt und Bestrahlungsabschnitt
- 1002: herkömmlicher Anpassungsabschnitt
- 1004: herkömmlicher Bestrahlungsabschnitt

- 200: Soll-Strahlführungsbahn
- 202: Ist-Strahlführungsbahn
- 204: Differenz der Ist-Strahlführungsbahn und Soll-Strahlführungsbahn

- 2000: Grenze zwischen Anpassungsabschnitt und Bestrahlungsabschnitt
- 2002: Anpassungsabschnitt
- 2004: Bestrahlungsabschnitt

- 300: Soll-Strahlführungsbahn
- 302: Ist-Strahlführungsbahn
- 304: Differenz der Ist-Strahlführungsbahn und Soll-Strahlführungsbahn
- 400, 500, 600, 700, 800: Soll-Strahlführungsbahn
- 402, 502, 602, 702, 802: Ist-Strahlführungsbahn
- 606, 706, 806: Anpassungsbewegung

- 3000: Grenze zwischen Anpassungsabschnitt und Bestrahlungsabschnitt
- 3002: Anpassungsabschnitt
- 3004: Bestrahlungsabschnitt
- 3006: weiterer Bestrahlungsabschnitt
- 3008: Unterbrechungsabschnitt

## Patentansprüche

1. Verfahren zur Bereitstellung einer Soll-Strahlführungsbahn (200) zur Ablenkung eines Laserstrahls (14) eines ophthalmologischen Lasersystems (10) mittels einer Verstelleinrichtung (16), das Verfahren umfassend:
- Festlegen eines Anpassungsabschnitts (2002) der Soll-Strahlführungsbahn (202), in welchem die Verstelleinrichtung (16) zur Strahlführung des Laserstrahls (14) aus dem Stillstand an einem Stillstandspunkt in eine kontinuierliche Bewegung überführt wird und in welchem keine Bestrahlung eines zu behandelnden Objektes mit dem Laserstrahl (14) vorgesehen ist;
- Festlegen eines Bestrahlungsabschnitts (2004) der Soll-Strahlführungsbahn (200), welcher an den Anpassungsabschnitt (2002) angrenzt, in welchem mittels der Verstelleinrichtung (16) der Laserstrahl (14) eine kontinuierliche Bewegung durchführt und in welchem eine Bestrahlung eines zu behandelnden Objektes mittels des Laserstrahls (14) vorgesehen ist;
charakterisiert dadurch, dass das Festlegen einer Länge und/oder Zeitdauer des Anpassungsabschnitts (2002) und/oder eines Abstands des Beginns des Bestrahlungsabschnitts (2004) vom Stillstandspunkt des Anpassungsabschnitts (2002) derart erfolgt, dass eine mit der Überführung der Verstelleinrichtung (16) aus dem Stillstand in die kontinuierliche Bewegung einhergehende Abweichung einer Ist-Strahlführungsbahn (202) von der festgelegten Soll-Strahlführungsbahn (200) im gesamten Bestrahlungsabschnitt (2004) kleiner als ein vorbestimmter Grenzwert ist.

2. Verfahren gemäß Anspruch 1, wobei das Festlegen einer Länge und/oder Zeitdauer des Anpassungsabschnitts (2002) und/oder eines Abstands des Bestrahlungsabschnitts (2004) vom Stillstandspunkt des Anpassungsabschnitts (2002) derart erfolgt, dass die Länge und/oder Zeitdauer zwischen dem Beginn des Überführens der Verstelleinrichtung (16) aus dem Stillstand in die kontinuierliche Bewegung und dem Beginn der vorgesehenen Bestrahlung des zu behandelnden Objekts gleich oder länger als eine vorbestimmte Sprungzeit und/oder gleich oder länger als ein vorbestimmter Sprungabstand bei der Überführung der Verstelleinrichtung (16) aus dem Stillstand in die kontinuierliche Bewegung ist.

3. Verfahren gemäß Anspruch 2, wobei die vorbestimmte Sprungzeit in einem Bereich von 1 ms bis 200 ms liegt und/oder wobei der vorbestimmte Sprungabstand bei der Überführung der Verstelleinrichtung (16) aus dem Stillstand in die kontinuierliche Bewegung in einem Bereich von 5 µm bis 500 µm liegt.

4. Verfahren gemäß Anspruch 2 oder 3, wobei das Festlegen eines Anpassungsabschnitts (2002) derart erfolgt, dass die Soll-Strahlführungsbahn (200) im Anpassungsabschnitt (2002) eine Anpassungsbewegung über eine Wegstrecke vorgibt, welche gleich oder länger als der Sprungabstand bei der Überführung der Verstelleinrichtung (16) aus dem Stillstand in die kontinuierliche Bewegung ist.

5. Verfahren gemäß Anspruch 4, wobei das Vorgeben der Anpassungsbewegung in dem Anpassungsabschnitt (2002) derart erfolgt, dass der Anpassungsabschnitt zeitlich reduziert wird; und/oder
wobei das Vorgeben der Anpassungsbewegung in dem Anpassungsabschnitt (2002) derart erfolgt, dass Abweichungen zwischen einer Ist-Strahlführungsbahn (202) und der Soll-Strahlführungsbahn 200 in kürzerer Zeit abgebaut werden als ohne die Anpassungsbewegung.

6. Verfahren gemäß einem der Ansprüche 4 bis 5, wobei die Anpassungsbewegung eine oder mehrere der folgenden Bewegungen aufweist:
- eine Bewegung der Verstelleinrichtung mit einer maximalen von der Verstelleinrichtung ausführbaren Beschleunigung;
- eine sprunghafte Bewegung der Verstelleinrichtung;
- eine Bewegung entlang eines nicht-differenzierbaren Abschnitts der Soll-Strahlführungsbahn;
- eine Bewegung, welche zwischen einem Stillstandspunkt und dem Beginn des darauffolgenden Strahlungsabschnitts sowohl eine Beschleunigung als auch ein Verzögern der Verstelleinrichtung entlang zumindest einer Bewegungsrichtung beinhaltet.

7. Verfahren gemäß einem der Ansprüche 4 bis 6, wobei das Vorgeben der Anpassungsbewegung in dem Anpassungsabschnitt (2002) derart erfolgt, dass der Verlauf der Soll-Strahlführungsbahn zwischen einem Stillstandspunkt und dem Beginn des darauffolgenden Strahlungsabschnitts einen Vorzeichenwechsel der zweiten zeitlichen Ableitung der Position der Verstelleinrichtung (16) aufweist.

8. Verfahren gemäß einem der vorhergehenden Ansprüche, wobei das Festlegen der Länge und/oder Zeitdauer des Anpassungsabschnitts (2002) und/oder des Abstands des Beginns des Bestrahlungsabschnitts (2004) vom Stillstandspunkt des Anpassungsabschnitts (2002) ferner derart erfolgt, dass die Länge und/oder Zeitdauer des Anpassungsabschnitts (2002) und/oder der Abstand des Beginns des Bestrahlungsabschnitts (2004) vom Stillstandspunkt minimal ist.

9. Verfahren gemäß einem der vorhergehenden Ansprüche, wobei die Soll-Strahlführungsbahn (200) mehrere Bestrahlungsabschnitte (2004, 3004, 3006) und/oder mehrere Anpassungsabschnitte (2002, 3002) aufweist, und/oder wobei die Soll-Strahlführungsbahn (200) zumindest zwei Bestrahlungsabschnitte (2004, 3004, 3006) aufweist und zumindest einen Anpassungsabschnitt (2002, 3002), welcher sich zwischen den beiden Bestrahlungsabschnitten erstreckt.

10. Verfahren gemäß einem der vorhergehenden Ansprüche, wobei der Anpassungsabschnitt (2002) und/oder der Bestrahlungsabschnitt (2004) ein Überführen der Verstelleinrichtung (16) aus einer kontinuierlichen Bewegung in den Stillstand umfasst und das Überführen der Verstelleinrichtung (16) aus dem Stillstand in eine kontinuierliche Bewegung im Anpassungsabschnitt (2002) zeitlich nach der Überführung der Verstelleinrichtung (16) aus der kontinuierlichen Bewegung in den Stillstand erfolgt.

11. Verfahren gemäß einem der vorhergehenden Ansprüche, ferner umfassend
- ein Ermitteln, ob bei einer Überführung der Verstelleinrichtung (16) zur Strahlführung des Laserstrahls (14) aus dem Stillstand an einem bestimmten Stillstandspunkt in eine kontinuierliche Bewegung die damit einhergehende Abweichung der Ist-Strahlführungsbahn (302) von der festgelegten Soll-Strahlführungsbahn (300) den vorbestimmten Grenzwert überschreitet, wobei der vorbestimmte Grenzwert optional in Abhängigkeit eines Positionsfehlers bestimmt ist, bei welchem bei Auftreten während eines Bestrahlungsabschnitts ein Abbruch der Bestrahlung des zu behandelnden Objektes mittels des Laserstrahls (14) erfolgen würde;
- Festlegen des Anpassungsabschnitts (3002), sofern ein Überschreiten des vorbestimmten Grenzwerts aufgrund des bestimmten Stillstandspunkts ermittelt wird; und
- Bereitstellen eines Unterbrechungsabschnitts (3008), falls kein Überschreiten des vorbestimmten Grenzwerts aufgrund des bestimmten Stillstandspunkts ermittelt wird, wobei in dem Unterbrechungsabschnitt (3008) keine Bestrahlung des zu behandelnden Objektes mit dem Laserstrahl (14) und keine Maßnahme zur Verringerung der mit der Überführung der Verstelleinrichtung (16) aus dem Stillstand in die kontinuierliche Bewegung einhergehenden Abweichung der Ist-Strahlführungsbahn (302) von der festgelegten Soll-Strahlführungsbahn (300) vorgesehen ist.

12. Steuereinheit (22) zur Bereitstellung einer Soll-Strahlführungsbahn (200) zur Ablenkung eines Laserstrahls (14) eines ophthalmologischen Lasersystems (10) mittels einer Verstelleinrichtung (16), wobei die Steuereinheit (20) dazu eingerichtet ist
- einen Anpassungsabschnitt (2002) der Soll-Strahlführungsbahn (200) festzulegen, in welchem die Verstelleinrichtung (16) zur Strahlführung des Laserstrahls (14) aus dem Stillstand an einem Stillstandspunkt in eine kontinuierliche Bewegung überführt wird und in welchem keine Bestrahlung eines zu behandelnden Objektes mit dem Laserstrahl (14) vorgesehen ist;
- einen Bestrahlungsabschnitt (2004) der Soll-Strahlführungsbahn (200) festzulegen, welcher an den Anpassungsabschnitt (2002) angrenzt, in welchem mittels der Verstelleinrichtung (16) der Laserstrahl (14) eine kontinuierliche Bewegung durchführt und in welchem eine Bestrahlung eines zu behandelnden Objektes mittels des Laserstrahls (14) vorgesehen ist;
charakterisiert dadurch, dass die Steuereinheit (22) weiterhin dazu eingerichtet ist, eine Länge und/oder Zeitdauer des Anpassungsabschnitts (2002) und/oder eines Abstands des Beginns des Bestrahlungsabschnitts (2004) vom Stillstandspunkt des Anpassungsabschnitts (2002) derart festzulegen, dass eine mit der Überführung der Verstelleinrichtung (16) aus dem Stillstand in die kontinuierliche Bewegung einhergehende Abweichung einer Ist-Strahlführungsbahn (202) von der festgelegten Soll-Strahlführungsbahn (200) im gesamten Bestrahlungsabschnitt (2004) kleiner als ein vorbestimmter Grenzwert ist.

13. Ophthalmologisches Lasersystem (10) umfassend:
- eine Laserquelle (12) zur Bereitstellung eines Laserstrahls (14) zur Behandlung eines zu behandelnden Objektes;
- eine Verstelleinrichtung (16) zur Strahlführung des Laserstrahls (14) gemäß einer vorgegebenen Soll-Strahlführungsbahn (200), wobei die Verstelleinrichtung (16) bei einer Überführung aus dem Stillstand in eine kontinuierliche Bewegung einen Losbrechsprung verursacht;
- eine Steuereinheit (22) zur Festlegung der Soll-Strahlführungsbahn (200) zur Bereitstellung für die Verstelleinrichtung (16), wobei die Steuereinheit (22) dazu eingerichtet ist:
+ einen Anpassungsabschnitt (2002) der Soll-Strahlführungsbahn (200) festzulegen, in welchem die Verstelleinrichtung (16) zur Strahlführung des Laserstrahls (14) aus dem Stillstand an einem Stillstandspunkt in eine kontinuierliche Bewegung überführt wird und in welchem keine Bestrahlung eines zu behandelnden Objektes mit dem Laserstrahl (14) erfolgt;
+ einen Bestrahlungsabschnitt (2004) der Soll-Strahlführungsbahn (200) festzulegen, welcher an den Anpassungsabschnitt (2004) angrenzt, in welchem mittels der Verstelleinrichtung (16) der Laserstrahl (14) eine kontinuierliche Bewegung durchführt und in welchem eine Bestrahlung des zu behandelnden Objektes mit dem Laserstrahl (14) erfolgt;
- eine Überwachungseinheit zur Ermittlung einer Ist-Strahlführungsbahn (202) entlang welcher die Strahlführung des Laserstrahls (14) mittels der Verstelleinrichtung (16) erfolgt;
dadurch charakterisiert, dass das ophthalmologische Lasersystem (10) dazu eingerichtet ist, eine mit dem Losbrechsprung einhergehende Abweichung der Ist-Strahlführungsbahn (202) von der Soll-Strahlführungsbahn (200) zu ermitteln und eine Länge und/oder Zeitdauer des Anpassungsabschnitts (2002) und/oder eines Abstands des Beginns des Bestrahlungsabschnitts (2004) vom Anpassungsabschnitt (2002) derart festzulegen, dass die Abweichung der Ist-Strahlführungsbahn (202) von der Soll-Strahlführungsbahn (200) im gesamten Bestrahlungsabschnitt (2004) kleiner als ein vorbestimmter Grenzwert ist.

14. Ophthalmologisches Lasersystem (10) gemäß Anspruch 13, wobei die Steuereinheit (22) dazu eingerichtet ist, die Verstelleinrichtung (16) zu steuern und/oder zu regeln; und
wobei die Verstelleinrichtung (16) optional als Linearversteller für ein Verstellen einer Fokuslage entlang der optischen Achse des Laserstrahls (14) ausgebildet ist.

15. Computerprogrammprodukt, umfassend Befehle, die bei der Ausführung des Programms durch eine Recheneinheit diese veranlassen, ein Verfahren gemäß einem der Ansprüche 1 bis 11 auszuführen.

## Claims

1. Method for providing a target beam guidance path (200) for deflecting a laser beam (14) of an ophthalmological laser system (10) by means of an adjusting device (16), the method comprising:
- defining an adaptation section (2002) of the target beam guidance path (202), in which the adjusting device (16), for beam guidance of the laser beam (14), is taken from a standstill at a standstill point to a continuous movement and in which there is no provision for an object that is to be treated to be irradiated with the laser beam (14);
- defining an irradiation section (2004) of the target beam guidance path (200), adjoining the adaptation section (2002), in which, by means of the adjusting device (16), the laser beam (14) performs a continuous movement and in which there is provision for an object that is to be treated to be irradiated by means of the laser beam (14);
**characterized in that**
a length and/or duration of the adaptation section (2002) and/or a distance of the start of the irradiation section (2004) from the standstill point of the adaptation section (2002) are/is defined in such a way that a variance between an actual beam guidance path (202) and the defined target beam guidance path (200) that is associated with the adjusting device (16) being taken from a standstill to the continuous movement is less than a predetermined limit value throughout the irradiation section (2004).

2. Method according to Claim 1, wherein a length and/or duration of the adaptation section (2002) and/or a distance of the irradiation section (2004) from the standstill point of the adaptation section (2002) are/is defined in such a way that the length and/or duration between the start of the adjusting device (16) being taken from a standstill to the continuous movement and the start of the intended irradiation of the object that is to be treated is equal to or longer than a predetermined jump time and/or equal to or longer than a predetermined jump distance when the adjusting device (16) is taken from a standstill to the continuous movement.

3. Method according to Claim 2, wherein the predetermined jump time is in a range from 1 ms to 200 ms and/or wherein the predetermined jump distance when the adjusting device (16) is taken from a standstill to the continuous movement is in a range from 5 µm to 500 µm.

4. Method according to Claim 2 or 3, wherein an adaptation section (2002) is defined in such a way that the target beam guidance path (200) specifies an adaptation movement in the adaptation section (2002) over a distance that is equal to or longer than the jump distance when the adjusting device (16) is taken from a standstill to the continuous movement.

5. Method according to Claim 4, wherein the adaptation movement in the adaptation section (2002) is specified in such a way that the adaptation section is reduced in time; and/or
wherein the adaptation movement in the adaptation section (2002) is specified in such a way that variances between an actual beam guidance path (202) and the target beam guidance path (200) are reduced in a shorter time than without the adaptation movement.

6. Method according to either of Claims 4 to 5, wherein the adaptation movement comprises one or more of the following movements:
- a movement of the adjusting device at a maximum acceleration that can be carried out by the adjusting device;
- a sudden movement of the adjusting device;
- a movement along a non-differentiable section of the target beam guidance path;
- a movement that includes both an acceleration and a deceleration of the adjusting device in at least one direction of movement between a standstill point and the start of the subsequent radiation section.

7. Method according to one of Claims 4 to 6, wherein the adaptation movement in the adaptation section (2002) is specified in such a way that the course of the target beam guidance path between a standstill point and the start of the subsequent radiation section has a change of arithmetic sign in the second time derivative of the position of the adjusting device (16).

8. Method according to one of the preceding claims, wherein the length and/or duration of the adaptation section (2002) and/or the distance of the start of the irradiation section (2004) from the standstill point of the adaptation section (2002) are/is also defined in such a way that the length and/or duration of the adaptation section (2002) and/or the distance of the start of the irradiation section (2004) from the standstill point is minimal.

9. Method according to one of the preceding claims, wherein the target beam guidance path (200) has multiple irradiation sections (2004, 3004, 3006) and/or multiple adaptation sections (2002, 3002); and/or wherein the target beam guidance path (200) has at least two irradiation sections (2004, 3004, 3006) and at least one adaptation section (2002, 3002) that extends between the two irradiation sections.

10. Method according to one of the preceding claims, wherein the adaptation section (2002) and/or the irradiation section (2004) comprises the adjusting device (16) being taken from a continuous movement to a standstill, and the adjusting device (16) is taken from a standstill to a continuous movement in the adaptation section (2002) at a time after the adjusting device (16) is taken from the continuous movement to a standstill.

11. Method according to one of the preceding claims, also comprising
- determining whether, when the adjusting device (16), for beam guidance of the laser beam (14), is taken from a standstill at a specific standstill point to a continuous movement, the associated variance between the actual beam guidance path (302) and the defined target beam guidance path (300) exceeds the predetermined limit value, the predetermined limit value optionally being determined according to a position error that, if it occurs during an irradiation section, would result in the irradiation of the object that is to be treated by means of the laser beam (14) being terminated;
- defining the adaptation section (3002) if the predetermined limit value is determined to have been exceeded on the basis of the specific standstill point; and
- providing an interruption section (3008) if the predetermined limit value is not determined to have been exceeded on the basis of the specific standstill point, there being no provision in the interruption section (3008) for the object that is to be treated to be irradiated with the laser beam (14) or for a measure for reducing the variance between the actual beam guidance path (302) and the defined target beam guidance path (300) that is associated with the adjusting device (16) being taken from a standstill to the continuous movement.

12. Control unit (22) for providing a target beam guidance path (200) for deflecting a laser beam (14) of an ophthalmological laser system (10) by means of an adjusting device (16), the control unit (20) being configured
- to define an adaptation section (2002) of the target beam guidance path (200), in which the adjusting device (16), for beam guidance of the laser beam (14), is taken from a standstill at a standstill point to a continuous movement and in which there is no provision for an object that is to be treated to be irradiated with the laser beam (14);
- to define an irradiation section (2004) of the target beam guidance path (200), adjoining the adaptation section (2002), in which, by means of the adjusting device (16), the laser beam (14) performs a continuous movement and in which there is provision for an object that is to be treated to be irradiated by means of the laser beam (14);
**characterized in that**
the control unit (22) is furthermore configured to define a length and/or duration of the adaptation section (2002) and/or a distance of the start of the irradiation section (2004) from the standstill point of the adaptation section (2002) in such a way that a variance between an actual beam guidance path (202) and the defined target beam guidance path (200) that is associated with the adjusting device (16) being taken from a standstill to the continuous movement is less than a predetermined limit value throughout the irradiation section (2004).

13. Ophthalmological laser system (10) comprising:
- a laser source (12) for providing a laser beam (14) for treating an object that is to be treated;
- an adjusting device (16) for beam guidance of the laser beam (14) along a predefined target beam guidance path (200), the adjusting device (16) causing a breakaway jump when taken from a standstill to a continuous movement;
- a control unit (22) for defining the target beam guidance path (200) to be provided for the adjusting device (16), the control unit (22) being configured:
+ to define an adaptation section (2002) of the target beam guidance path (200), in which the adjusting device (16), for beam guidance of the laser beam (14), is taken from a standstill at a standstill point to a continuous movement and in which an object that is to be treated is not irradiated with the laser beam (14);
+ to define an irradiation section (2004) of the target beam guidance path (200), adjoining the adaptation section (2004), in which, by means of the adjusting device (16), the laser beam (14) performs a continuous movement and in which the object that is to be treated is irradiated with the laser beam (14);
- a monitoring unit for determining an actual beam guidance path (202) along which the beam guidance of the laser beam (14) is performed by means of the adjusting device (16);
**characterized in that**
the ophthalmological laser system (10) is configured to determine a variance between the actual beam guidance path (202) and the target beam guidance path (200) that is associated with the breakaway jump and to define a length and/or duration of the adaptation section (2002) and/or a distance of the start of the irradiation section (2004) from the adaptation section (2002) in such a way that the variance between the actual beam guidance path (202) and the target beam guidance path (200) is less than a predetermined limit value throughout the irradiation section (2004).

14. Ophthalmological laser system (10) according to Claim 13, the control unit (22) being configured to perform open-loop and/or closed-loop control of the adjusting device (16); and
the adjusting device (16) optionally being in the form of a linear adjuster for adjusting a focal position along the optical axis of the laser beam (14).

15. Computer program product comprising instructions that, when the program is executed by a computing unit, cause said computing unit to carry out a method according to one of Claims 1 to 11.

## Revendications

1. Procédé pour fournir une voie de guidage de faisceau de consigne (200) pour dévier un faisceau laser (14) d'un système de laser ophtalmologique (10) à l'aide d'un dispositif de réglage (16), le procédé comprenant les étapes consistant à :
- fixer une partie d'adaptation (2002) de la voie de guidage de faisceau de consigne (202), étape au cours de laquelle on fait passer le dispositif de réglage (16) pour le guidage du faisceau laser (14) de l'arrêt en un point d'arrêt à un mouvement continu et au cours de laquelle aucun rayonnement n'est prévu pour un objet à traiter au moyen du faisceau laser (14) ;
- fixer une partie d'application de rayonnement (2004) de la voie de guidage de faisceau de consigne (200), qui est adjacente à la partie d'adaptation (2002), étape au cours de laquelle le faisceau laser (14) exécute un mouvement continu à l'aide du dispositif de réglage (16) et au cours de laquelle une application de rayonnement à un objet à traiter est prévue au moyen du faisceau laser (14) ;
**caractérisé en ce que**
l'établissement d'une longueur et/ou d'une durée de la partie d'adaptation (2002) et/ou d'une distance entre le début de la partie d'application de rayonnement (2004) et le point d'arrêt de la partie d'adaptation (2002) est effectué de telle sorte qu'un écart d'une voie de guidage de faisceau réelle (202) par rapport à la voie de guidage de faisceau de consigne (200) fixée, écart qui accompagne le passage du dispositif de réglage (16) de l'arrêt au mouvement continu, soit inférieur à une valeur limite prédéterminée dans la totalité de la partie d'application de rayonnement (2004).

2. Procédé selon la revendication 1, dans lequel l'établissement d'une longueur et/ou d'une durée de la partie d'adaptation (2002) et/ou d'une distance entre la partie d'application de rayonnement (2004) et le point d'arrêt de la partie d'adaptation (2002) est effectué de telle sorte que la longueur et/ou la durée entre le début du passage du dispositif de réglage (16) de l'arrêt au mouvement continu et le début de l'application de rayonnement prévue de l'objet à traiter soit égale ou supérieure à une durée de saut prédéterminée et/ou égale ou supérieure à une distance de saut prédéterminée lors du passage du dispositif de réglage (16) de l'arrêt au mouvement continu.

3. Procédé selon la revendication 2, dans lequel la durée de saut prédéterminée se situe dans une plage allant de 1 ms à 200 ms et/ou dans lequel la distance de saut prédéterminée lors du passage du dispositif de réglage (16) de l'arrêt au mouvement continu se situe dans une plage allant de 5 µm à 500 µm.

4. Procédé selon la revendication 2 ou 3, dans lequel l'établissement d'une partie d'adaptation (2002) est effectué de telle sorte que la voie de guidage de faisceau de consigne (200) dans la partie d'adaptation (2002) prescrit un mouvement d'adaptation sur une distance qui est égale ou supérieure à la distance de saut lors du passage du dispositif de réglage (16) de l'arrêt au mouvement continu.

5. Procédé selon la revendication 4, dans lequel la prescription du mouvement d'adaptation dans la partie d'adaptation (2002) est effectuée de telle sorte que la partie d'adaptation soit réduite temporellement ; et/ou dans lequel la prescription du mouvement d'adaptation dans la partie d'adaptation (2002) est effectuée de telle sorte que les écarts entre une voie de guidage de faisceau réelle (202) et la voie de guidage de faisceau de consigne (200) soient éliminés en un temps plus court qu'en l'absence du mouvement d'adaptation.

6. Procédé selon l'une quelconque des revendications 4 à 5, dans lequel le mouvement d'adaptation comprend un ou plusieurs des mouvements suivants :
- un mouvement du dispositif de réglage avec une accélération maximale pouvant être produite par le dispositif de réglage ;
- un mouvement de saut du dispositif de réglage ;
- un mouvement le long d'une partie non différenciable de la voie de guidage de faisceau de consigne ;
- un mouvement qui inclut à la fois une accélération et une décélération du dispositif de réglage le long d'au moins une direction de mouvement entre un point d'arrêt et le début de la partie d'application de rayonnement suivante.

7. Procédé selon l'une quelconque des revendications 4 à 6, dans lequel la prescription du mouvement d'adaptation dans la partie d'adaptation (2002) est effectuée de telle sorte que le tracé de la voie de guidage de faisceau de consigne entre un point d'arrêt et le début de la partie d'application de rayonnement suivante présente un changement de signe de la dérivée seconde par rapport au temps de la position du dispositif de réglage (16).

8. Procédé selon l'une quelconque des revendications précédentes, dans lequel l'établissement de la longueur et/ou de la durée de la partie d'adaptation (2002) et/ou de la distance entre le début de la partie d'application de rayonnement (2004) et le point d'arrêt de la partie d'adaptation (2002) est en outre effectué de telle sorte que la longueur et/ou la durée de la partie d'adaptation (2002) et/ou la distance entre le début de la partie d'application de rayonnement (2004) et le point d'arrêt soit minimal.

9. Procédé selon l'une quelconque des revendications précédentes, dans lequel la voie de guidage de faisceau de consigne (200) comporte plusieurs parties d'application de rayonnement (2004, 3004, 3006) et/ou plusieurs parties d'adaptation (2002, 3002) ; et/ou dans lequel la voie de guidage de faisceau de consigne (200) comporte au moins deux parties d'application de rayonnement (2004, 3004, 3006) et au moins une partie d'adaptation (2002, 3002) qui s'étend entre les deux parties d'application de rayonnement.

10. Procédé selon l'une quelconque des revendications précédentes, dans lequel la partie d'adaptation (2002) et/ou la partie d'application de rayonnement (2004) comprend un passage du dispositif de réglage (16) d'un mouvement continu à l'arrêt, et le passage du dispositif de réglage (16) de l'arrêt à un mouvement continu dans la partie d'adaptation (2002) s'effectue après le passage du dispositif de réglage (16) du mouvement continu à l'arrêt.

11. Procédé selon l'une quelconque des revendications précédentes, comprenant en outre les étapes consistant à
- déterminer si, lors d'un passage du dispositif de réglage (16) pour le guidage du faisceau laser (14) de l'arrêt en un certain point d'arrêt à un mouvement continu, l'écart qui l'accompagne de la voie de guidage de faisceau réelle (302) par rapport à la voie de guidage de faisceau de consigne (300) fixée dépasse la valeur limite prédéterminée, la valeur limite prédéterminée étant facultativement déterminée en fonction d'une erreur de position au cours de laquelle, lorsque cela se produit pendant une partie d'application de rayonnement, une interruption de l'application de rayonnement de l'objet à traiter au moyen du faisceau laser (14) a lieu ;
- fixer la partie d'adaptation (3002), à condition qu'un dépassement de la valeur limite prédéterminée soit déterminé en raison du point d'arrêt déterminé ; et
- fournir une partie d'interruption (3008), si aucun dépassement de la valeur limite prédéterminée n'est déterminé en raison du point d'arrêt déterminé, aucune application de rayonnement à l'objet à traiter n'étant prévue au moyen du faisceau laser (14) dans la partie d'interruption (3008) et aucune mesure n'étant prévue pour réduire l'écart de la voie de guidage de faisceau réelle (302) par rapport à la voie de guidage de faisceau de consigne (300) fixée, lequel écart accompagne le passage du dispositif de réglage (16) de l'arrêt au mouvement continu.

12. Unité de commande (22) destinée à fournir une voie de guidage de faisceau de consigne (200) pour dévier un faisceau laser (14) d'un système de laser ophtalmologique (10) au moyen d'un dispositif de réglage (16), l'unité de commande (20) étant conçue pour
- fixer une partie d'adaptation (2002) de la voie de guidage de faisceau de consigne (200), étape au cours de laquelle on fait passer le dispositif de réglage (16) pour le guidage du faisceau laser (14) de l'arrêt en un point d'arrêt à un mouvement continu et au cours de laquelle aucun rayonnement n'est prévu pour un objet à traiter au moyen du faisceau laser (14) ;
- fixer une partie d'application de rayonnement (2004) de la voie de guidage de faisceau de consigne (200), qui est adjacente à la partie d'adaptation (2002), étape au cours de laquelle le faisceau laser (14) exécute un mouvement continu à l'aide du dispositif de réglage (16) et au cours de laquelle une application de rayonnement à un objet à traiter est prévue au moyen du faisceau laser (14) ;
**caractérisé en ce que**
l'unité de commande (22) est en outre conçue pour établir une longueur et/ou une durée de la partie d'adaptation (2002) et/ou une distance entre le début de la partie d'application de rayonnement (2004) et le point d'arrêt de la partie d'adaptation (2002) de telle sorte qu'un écart d'une voie de guidage de faisceau réelle (202) par rapport à la voie de guidage de faisceau de consigne (200) fixée, écart qui accompagne le passage du dispositif de réglage (16) de l'arrêt au mouvement continu, soit inférieur à une valeur limite prédéterminée dans la totalité de la partie d'application de rayonnement (2004).

13. Système de laser ophtalmologique (10) comprenant :
- une source laser (12) pour fournir un faisceau laser (14) pour le traitement d'un objet à traiter ;
- un dispositif de réglage (16) pour le guidage du faisceau laser (14) suivant une voie de guidage de faisceau de consigne (200) donnée, le dispositif de réglage (16) provoquant un saut de séparation lors d'un passage de l'arrêt à un mouvement continu ;
- une unité de commande (22) pour établir la voie de guidage de faisceau de consigne (200) en vue de sa fourniture au dispositif de réglage (16), l'unité de commande (22) étant conçue pour :
+ fixer une partie d'adaptation (2002) de la voie de guidage de faisceau de consigne (200), étape au cours de laquelle on fait passer le dispositif de réglage (16) pour le guidage du faisceau laser (14) de l'arrêt en un point d'arrêt à un mouvement continu et au cours de laquelle aucun rayonnement n'est prévu pour un objet à traiter au moyen du faisceau laser (14) ;
+ fixer une partie d'application de rayonnement (2004) de la voie de guidage de faisceau de consigne (200), qui est adjacente à la partie d'adaptation (2004), étape au cours de laquelle le faisceau laser (14) exécute un mouvement continu à l'aide du dispositif de réglage (16) et au cours de laquelle une application de rayonnement à un objet à traiter est effectuée au moyen du faisceau laser (14) ;
- une unité de surveillance destinée à déterminer une voie de guidage de faisceau réelle (202) le long de laquelle le guidage du faisceau laser (14) est effectué au moyen du dispositif de réglage (16) ;
**caractérisé en ce que**
le système de laser ophtalmologique (10) est conçu pour déterminer un écart de la voie de guidage de faisceau réelle (202) par rapport à la voie de guidage de faisceau de consigne (200) qui accompagne le saut de séparation et pour fixer une longueur et/ou une durée de la partie d'adaptation (2002) et/ou une distance entre le début de la partie d'application de rayonnement (2004) et la partie d'adaptation (2002) de telle sorte que l'écart de la voie de guidage de faisceau réelle (202) par rapport à la voie de guidage de faisceau de consigne (200) soit inférieur à une valeur limite prédéterminée dans la totalité de la partie d'application de rayonnement (2004).

14. Système de laser ophtalmologique (10) selon la revendication 13, dans lequel l'unité de commande (22) est conçue pour commander et/ou réguler le dispositif de réglage (16) ; et
dans lequel le dispositif de réglage (16) est facultativement réalisé sous forme de dispositif de réglage linéaire pour un réglage d'une position focale le long de l'axe optique du faisceau laser (14).

15. Programme informatique comprenant des instructions qui, lors de l'exécution du programme par une unité de calcul, amènent ce dernier à mettre en œuvre un procédé selon l'une quelconque des revendications 1 à 11.
